# FASCICULE DE BREVET EUROPEEN

(11) **EP 0 571 243 B1**
(45) Date de publication et mention de la délivrance du brevet: **26.07.1995**
(21) Numéro de dépôt: 93401193.3
(22) Date de dépôt: 11.05.1993
(51) Int. Cl.: C07D 409/12, C07D 413/12, A61K 31/395

(54) **Nouveaux composés thiochromaniques, leurs procédés de préparation et les compositions pharmaceutiques qui les contiennent**
Thiochromanderivate, Verfahren zu ihrer Herstellung und diese enthaltende pharmazeutische Zubereitungen
Thiochromane derivatives, process for their preparation and pharmaceutical compositions containing them

(30) Priorité: 18.05.1992 FR 9205960
(43) Date de publication de la demande: 24.11.1993
(73) Titulaire: ADIR ET COMPAGNIE, 92415 Courbevoie Cédex (FR)
(72) Inventeur: Guillaumet, Gérald, F-45100 Orleans (FR); Coudert, Gérard, F-45100 Orleans (FR); Podona, Tchao, F-75017 Paris (FR); Guardiola-Lemaitre, Béatrice, F-92200 Neuilly sur Seine (FR); Renard, Pierre, F-78000 Versailles (FR); Adam, Gérard, F-78600 Le Mesnil le Roi (FR); Caignard, Daniel Henri, F-75015 Paris (FR)

(56) Documents cités:
- EP-A- 0 458 459
- WO-A-91/09006
- FR-A- 2 092 004
- US-A- 4 992 465

## Description

La présente invention concerne de nouveaux composés thiochromaniques, leur procédé de préparation, et les compositions pharmaceutiques qui les contiennent.

Certains dérivés thiochromaniques de formule (a) : 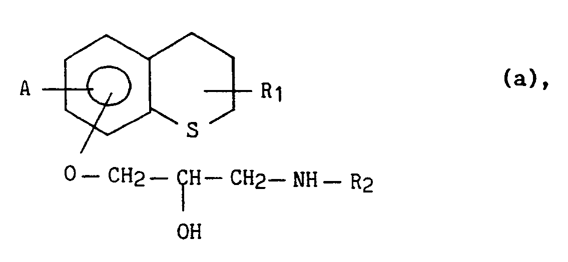
ont déjà été décrits en tant qu'antagonistes des récepteurs β adrénergiques (FR 209 20 04) et se sont révélés très utiles dans le traitement de l'hypertension artérielle.

D'autres dérivés thiochromaniques de formule (b) : 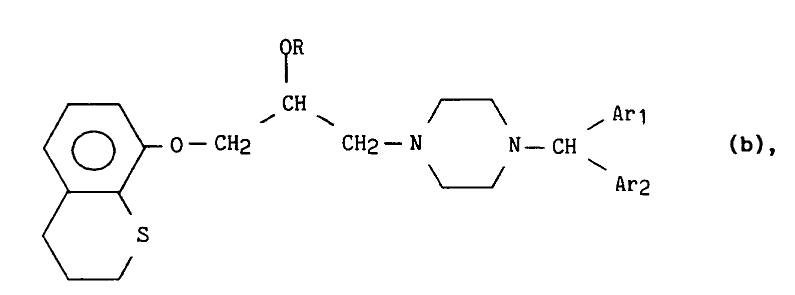
sont décrits dans la demande de brevet EP 458 459 et sont revendiqués pour leur activité cardiotonique.

On connait également un composé (pipéridinoéthyloxy)thiochromanique possédant également des propriétés cardiotoniques et décrit dans le brevet US 3,705,167.

La demanderesse a découvert de nouveaux composés thiochromaniques qui présentent de façon surprenante une très forte affinité pour les récepteurs sérotoninergiques.

Ils possèdent notamment un intense pouvoir antagoniste des récepteurs 5HT_{1A}.

D'autre part, ils se révèlent, de façon surprenante par rapport à l'art antérieur, peu affins pour les récepteurs β₁ et β₂.

Ces caractéristiques les rendent utilisables en thérapie, dans les troubles du système nerveux central (anxiété, dépression, stress, psychoses, schizophrénie, douleur et troubles du comportement alimentaire et sexuel), avec une absence d'effets secondaires au niveau du système cardiovasculaire, puisque les composés de l'invention n'ont qu'une faible affinité pour les récepteurs β, contrairement aux composés les plus proches de l'art antérieur.

Les indications des composés de l'invention diffèrent donc totalement de celles - cardiovasculaires - des dérivés thiochromaniques de l'art antérieur.

Plus spécifiquement, la présente invention a pour objet les composés de formule (I) : 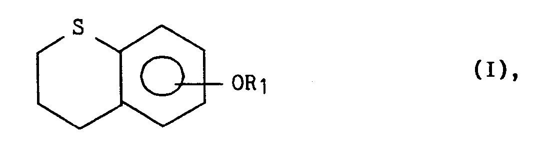
dans laquelle :
- R₁ représente :
   * un groupement de formule (A) : 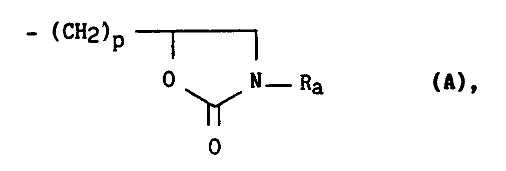 dans laquelle Rₐ représente un atome d'hydrogène ou un radical alkyle inférieur et p est égal à 1, 2 ou 3,
   * ou un groupement de formule (B) : 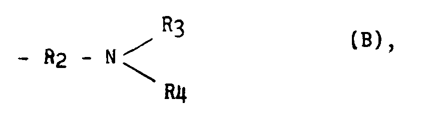 dans laquelle
      → R₂ est un groupement choisi parmi :
         - -R'₂- où R'₂ représente un groupement -(CH₂)ₙ- ou 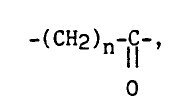 avec n représentant un nombre entier de 1 à 6, R'₂ étant non substitué ou substitué sur la partie alkylène par un radical alkyle inférieur, aryle, ou arylalkyle inférieur,
         - et 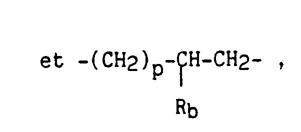 où p est égal à 1, 2 ou 3 et où R_{b} représente un radical hydroxyle, alkoxy inférieur, alkylcarbonyloxy inférieur, aryloxy, ou (arylalkyle inférieur)oxy,
      → R₃ et R₄, identiques ou différents, représentent, chacun indépendamment l'un de l'autre,
         - un atome d'hydrogène,
         - un radical alkyle inférieur, non substitué ou substitué par un ou plusieurs radicaux choisis parmi halogène, hydroxyle et alcoxy inférieur,
         - un radical cycloalkyle, cycloalkylalkyle inférieur, di(cycloalkyl)-alkyle inférieur, non substitué ou substitué sur les parties "cycloalkyle" par un ou plusieurs radicaux choisis parmi halogène, oxo, alkyle inférieur et alkoxy inférieur,
         - un radical acyle, non substitué ou substitué par un ou plusieurs radicaux choisis parmi halogène, alkyle inférieur et alkoxy inférieur,
         - un radical choisi parmi adamantyle, benzopyranyle, benzofuryle, et aryle ; ce radical étant non substitué ou substitué par un ou plusieurs radicaux choisis parmi halogène, hydroxyle, alkyle inférieur, alkoxy inférieur et trifluorométhyle,
         - un radical arylalkyle inférieur, non substitué ou substitué sur le noyau aryle par un ou plusieurs radicaux choisis parmi halogène, hydroxyle, alkyle inférieur, alkoxy inférieur, et trifluorométhyle,
         - un radical 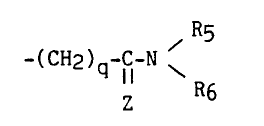 dans lequel q représente 0 ou un nombre entier de 1 à 4 et Z représente un atome d'oxygène ou 2 atomes d'hydrogène,
            et dans lesquels R₅ et R₆ représentent, chacun indépendamment l'un de l'autre, un atome d'hydrogène, un alkyle inférieur, un acyle, un aryle non substitué ou substitué par un ou plusieurs radicaux choisis parmi halogène, hydroxyle, alkyle inférieur, alkoxy inférieur, et trifluorométhyle ; ou un arylalkyle inférieur non substitué ou substitué sur le noyau aryle par un ou plusieurs radicaux choisis parmi halogène, hydroxyle, alkyle inférieur, alkoxy inférieur, et trifluorométhyle,
            ou R₅ et R₆ forment ensemble avec l'atome d'azote qui les porte soit un hétérocycle, non substitué ou substitué, choisi parmi pyrrolidine, pipéridine, pipérazine, morpholine, thiomorpholine, homopipérazine, homopipéridine, phtalimide, et azabicycloalkyle contenant de 7 à 12 sommets, soit un groupement (azaspiroalkyl)alkyle inférieur, le système azaspiroalkyle comprenant de 6 à 12 sommets, et étant non substitué ou substitué par un ou plusieurs radicaux choisis parmi halogène, oxo, et alkyle inférieur,
            avec la réserve que lorsque R₂ représente un groupement 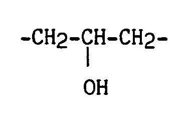 et simultanément R₃ est un atome d'hydrogène, alors R₄ ne peut pas représenter un atome d'hydrogène, un radical alkyle inférieur, ou un radical cycloalkyle,
      → ou R₃ et R₄ forment avec l'atome d'azote qui les porte :
         - soit un hétérocycle, non substitué ou substitué, choisi parmi pyrrolidine, pipéridine, pipérazine, morpholine, thiomorpholine, homopipérazine, homopipéridine, phtalimide, et azabicycloalkyle contenant de 7 à 12 sommets,
            avec la réserve que si R₂ représente un groupement -(CH₂)ₙ- avec n tel que défini précédemment alors R₃ et R₄ ne peuvent pas former avec l'atome d'azote qui les porte un groupement pipéridino non substitué ou substitué par un ou plusieurs radicaux alkyles inférieurs ou alkoxy inférieurs,
         - soit un groupement azaspiroalkyle de 6 à 12 sommets, non substitué ou substitué par un ou plusieurs radicaux choisis parmi halogène, oxo, alkyle inférieur, et alkoxy inférieur, étant entendu que lorsque R₂ représente un groupement 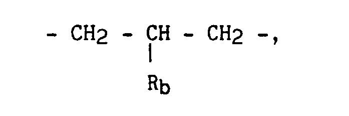 R₃ et R₄ ne peuvent pas former avec l'atome d'azote qui les porte une pipérazine substituée en position 4 par un radical diphénylméthyle non substitué ou substitué sur les noyaux phényles par un ou plusieurs radicaux choisis parmi halogène, alkyle inférieur, alkoxy inférieur et trifluorométhyle,
étant entendu que, sauf précisions contraires :
. l'expression "substitué" affectée au terme "hétérocycle" signifie que l'hétérocycle est substitué par un ou plusieurs radicaux choisis parmi :
   - halogène,
   - hydroxyle,
   - oxo,
   - alkyle inférieur,
   - alkoxy inférieur,
   - alkoxycarbonyle inférieur,
   - et les groupements -(CH₂)_{n'}-E, 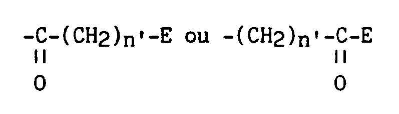 avec n' représentant 0 ou un nombre entier de 1 à 4 et E représentant un radical choisi parmi aryle, benzhydryle, thiényle, pyrrolyle, pyrrolidinyle, furyle, pyrimidinyle, pyridyle, cycloalkyle et dicycloalkylalkyle inférieur, le groupement E pouvant être non substitué ou substitué par un ou plusieurs groupements choisis parmi halogène, hydroxyle, alkyle inférieur, alkoxy inférieur, et trifluorométhyle,
. le terme "cycloalkyle" représente un groupement cyclique de 3 à 8 atomes de carbone,
. le terme "acyle" représente un groupement alkylcarbonyle inférieur, arylcarbonyle, et arylalkylcarbonyle inférieur,
. le terme "aryle" représente un groupement choisi parmi phényle et naphtyle,
. et les termes "alkyle inférieur" et "alkoxy inférieur" signifient des groupements, linéaires ou ramifiés, contenant de 1 à 6 atomes de carbone,
leurs isomères optiques, sous forme pure ou sous forme de mélange, ainsi que leurs sels d'addition à un acide ou une base pharmaceutiquement acceptable.

Plus particulièrement, R₃ et R₄ forment avec l'atome d'azote qui les porte une pipérazine non substituée ou substituée en position 4 par un radical choisi parmi :
- alkyle inférieur,
- alkoxy inférieur,
- alkoxycarbonyle inférieur
- et les groupements -(CH₂)_{n'}-E, 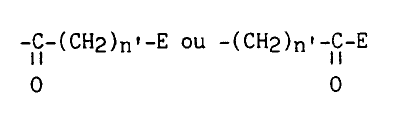 avec n' représentant 0 ou un nombre entier de 1 à 4 et E représentant un radical choisi parmi aryle, benzhydrile, thiényle, pyrrolyle, pyrrolidinyle, furyle, pyrimidinyle, pyridyle, cycloalkyle et dicycloalkylalkyle inférieur, le groupement E pouvant être non substitué ou substitué par un ou plusieurs groupements choisis parmi halogène, oxo, hydroxyle, alkyle inférieur, alkoxy inférieur, et trifluorométhyle.

Plus particulièrement, R₂ représente un groupement -(CH₂)ₙ- ou 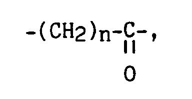
avec n repésentant un nombre entier de 1 à 6, les groupements -(CH₂)ₙ- et 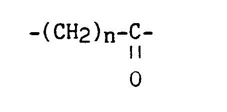
pouvant être substitués sur leur partie alkylène par un radical alkyle inférieur, aryle, ou arylalkyle inférieur.

Plus particulièrement, R₂ représente un groupement

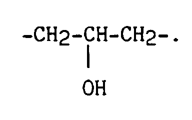

Parmi les acides utilisés pour la formation des sels d'additions des composés de formule (I), on peut citer, à titre d'exemples et de façon non limitative, dans la série minérale, les acides chlorhydrique, bromhydrique, sulfurique et phosphorique, et dans la série organique, les acides acétique, propionique, maléique, fumarique, tartrique, citrique, oxalique, benzoïque et méthanesulfonique.

Parmi les base pharmaceutiquement acceptables que l'on peut utiliser pour former un sel d'addition avec les composés de l'invention, on peut citer, à titre d'exemples et de façon non limitative, les hydroxydes de sodium, de potassium, de calcium, ou d'aluminium, la triéthylamine, la benzylamine, la diéthanolamine, la tert-butylamine, la dicyclohexylamine, et l'arginine.

L'invention s'étend également au procédé de préparation des composés de formule (I), caractérisé en ce que l'on utilise comme matière première un thiochromanol de formule (II) : 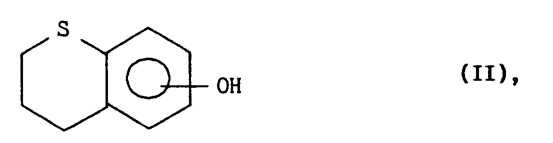
que l'on fait réagir :
- soit avec un composé di-halogéné de formule (IIa) :

   X₁-R'₂-Y'₁ **(II**_{**a**}**),**

   dans laquelle R'₂ est tel que défini dans la formule (I), X₁ représente un atome d'halogène et Y'₁ représente un atome d'halogène, ou un radical (C₁-C₄) alkoxy ou un hydroxyle lorsque R'₂ contient une fonction carbonyle, pour donner un composé de formule (II/b) : 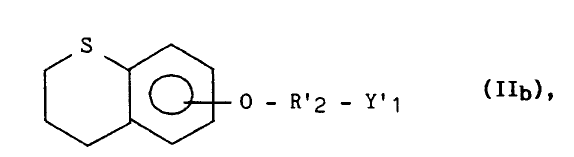 dans laquelle R'₂ et Y'₁ sont tels que définis précédemment, que l'on fait réagir, après une hydrolyse de la fonction ester lorsque Y₁ représente un radical (C₁-C₄)alkoxy, avec une amine de formule (II_{c}) : 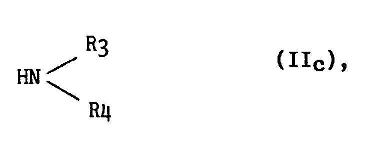 dans laquelle R₃ et R₄ sont tels que définis dans la formule (I), pour donner un composé de formule (I₁) : 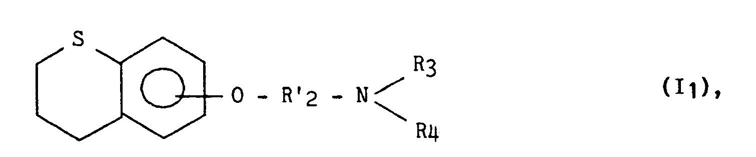 dans laquelle R'₂, R₃, et R₄ sont tels que définis précédemment,
   cas particulier des composés de formule (I) où R₁ représente un groupement 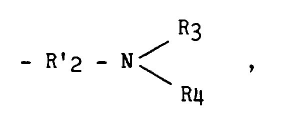 où R'₂, R₃ et R₄ sont tels que définis précédemment,
- soit avec un composé de formule (II_{d}) ou (II_{d'}) :

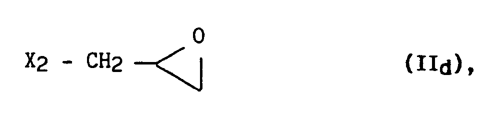

   X₂―(CH₂)ₚ―CH=CH₂ **(II**_{**d'**}**),**

   où X₂ représente un atome d'halogène, et où p est tel que défini dans la formule (I) pour donner, après traitement par un peracide lorsqu'on a utilisé un réactif de formule (II/_{d'}), un composé de formule (IIₑ): 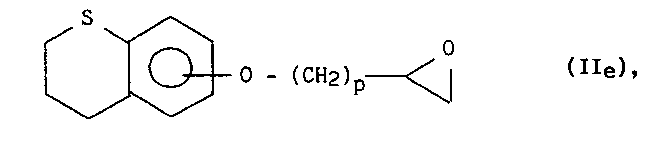 où p est tel que défini précédemment,
   composé de formule (IIe) que l'on fait réagir :
   * ou bien avec une amine de formule (II_{c}) : 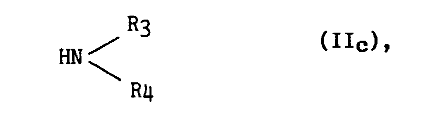 dans laquelle R₃ et R₄ sont tels que définis précédemment, pour donner un composé de formule (I₂) : 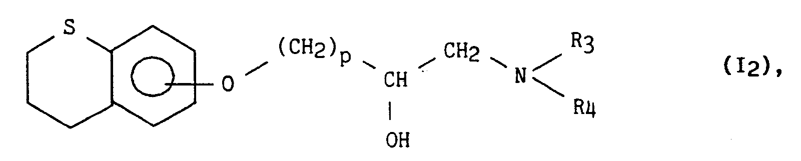 dans laquelle, p, R₃ et R₄ sont tels que définis précédemment, cas particulier des composés de formule (I), dans lequel R₁ représente un groupement : 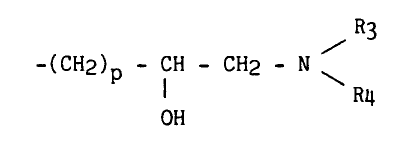 où p, R₃ et R₄ sont tels que définis précédemment,
      composés de formule (I₂) que l'on substitue sur le groupement hydroxyle situé en β de l'atome d'azote porteur des radicaux R₃ et R₄, par réaction avec un composé de formule (II_{f}) :

      X3 - R_{β} **(II**_{**f**}**)**

      dans lesquelles X₃ représente un atome d'halogène et R_{β} est un radical alkyle inférieur, alkylcarbonyl inférieur, aryle, ou arylalkyle inférieur avec les termes "alkyle inférieur", et "aryle" tels que définis dans la formule (I), réaction éventuellement accompagnée d'une étape de protection de l'azote portant R₃ et R₄, pour donner un produit de formule (I₃) : 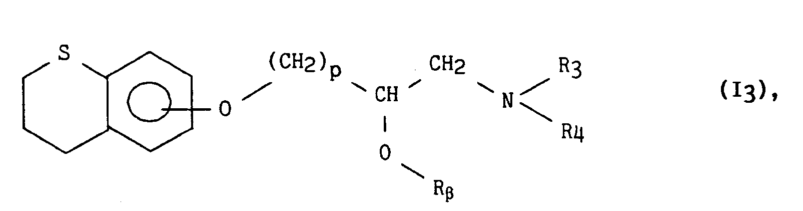 où p, R₃, R₄ et R_{β} sont tels que définis précédemment,
      cas particulier des composés de formule générale (I), où R₁ représente un groupement : 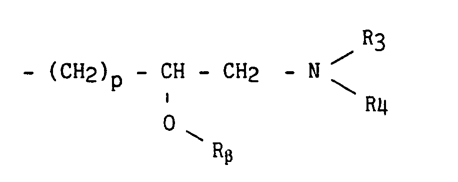 où p, R₃, R₄ et R_{β} sont tels que définis précédemment,
   * ou bien avec de l'azidure de sodium, pour donner un composé de formule (IIg) : 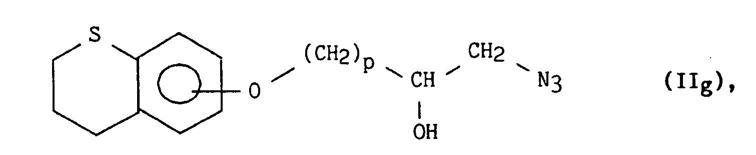 où p est tel que défini précédemment, que l'on fait ensuite réagir avec de l'éthanol et un catalyseur de Lindlar, afin d'obtenir une amine primaire de formule (I₄) : 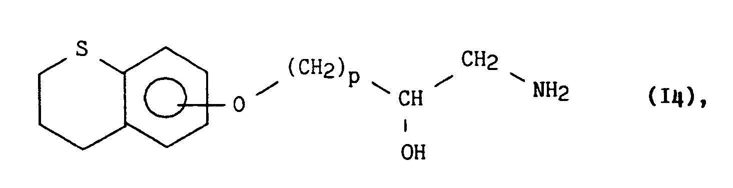 où p est tel que défini précédemment, cas particulier des composés de formule générale (I₂), où R₁ représente un groupement : 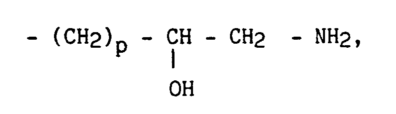 où p est tel que défini précédemment,
      composé de formule (I₄) que l'on fait réagir avec du N,N'-carbonyldiimidazole, pour obtenir un composé de formule (I₅) : 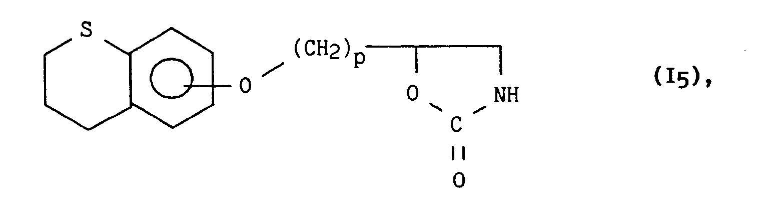 dans laquelle p est tel que défini précédemment, cas particulier des composés de formule (I) où R₁ représente un groupement de formule : 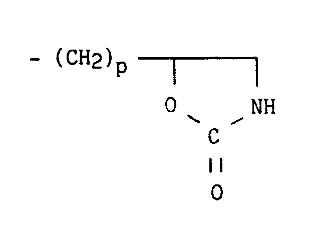 dans lequel p est tel que défini précédemment,
      que l'on alkyle, le cas échéant, par l'intermédiaire d'un halogénoalkyle inférieur de formule (IIₕ) :

      Rₐ' - X₄ **(II**_{**h**}**),**

      où Rₐ' est un radical alkyle inférieur et où X₄ est un atome d'halogène,
      pour donner un composé de formule (I₆) : 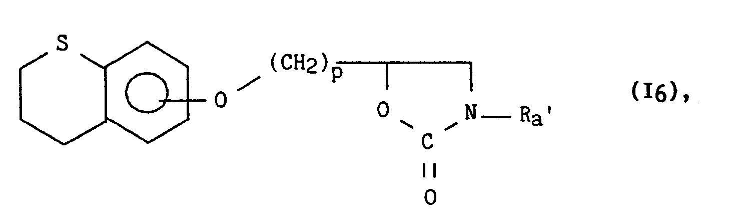 dans laquelle p et Rₐ' sont tels que définis précédemment,
      cas particulier des composés de formule (I) où R₁ représente un groupement de formule : 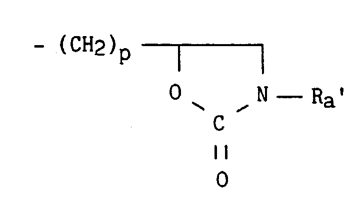 dans laquelle p et Rₐ' sont tels que définis précédemment,
   les composés de formule (I₁), (I₂), (I₃), (I₄), (I₅) et (I₆) constituent l'ensemble des composés de formule (I) qui peuvent être, si on le désire,
   - alkylés, si on est en présence d'une amine secondaire, sur l'azote portant les substituants R₃ et R₄,
   - purifiés suivant une ou plusieurs méthodes de purification choisies parmi la cristallisation, la chromatographie sur colonne de silice, l'extraction, la filtration, et le passage sur charbon ou résine,
   - séparés, le cas échéant, sous forme pure ou sous forme de mélange, en leurs éventuels isomères optiques,
   - ou salifiés, par un acide ou une base pharmaceutiquement acceptable.

La présente invention concerne également le procédé de préparation des composés de formule (I₇) et (I_{7'}) : 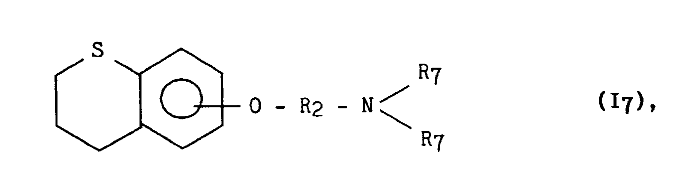 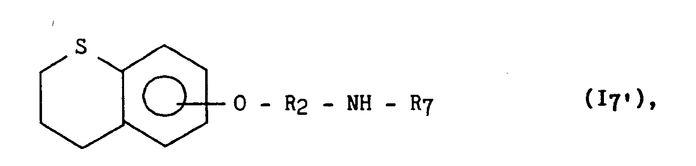
dans lesquelles R₂ est tel que défini dans la formule (I) et R₇ représente :
- un radical alkyle inférieur, non substitué ou substitué par un ou plusieurs radicaux choisis parmi halogène, hydroxyle et alcoxy inférieur,
- un radical cycloalkyle, cycloalkylalkyle inférieur, di(cycloalkyl)alkyle inférieur, non substitué ou substitué, sur les parties "cycloalkyle", par un ou plusieurs radicaux choisis parmi halogène, oxo, alkyle inférieur et alkoxy inférieur,
- un radical acyle, non substitué ou substitué par un ou plusieurs radicaux choisis parmi halogène, alkyle inférieur et alkoxy inférieur,
- un radical choisi parmi adamantyle, benzopyranyle, benzofuryle, et aryle, non substitué ou substitué par un ou plusieurs radicaux choisis parmi halogène, alkyle inférieur, alkoxy inférieur et trifluorométhyle,
- un radical arylalkyle inférieur, non substitué ou substitué sur le noyau aryle par un ou plusieurs radicaux choisis parmi halogène, alkyle inférieur, alkoxy inférieur et trifluorométhyle,
- ou un radical 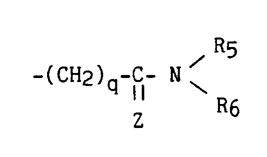 dans lequel q représente 0 ou un nombre entier de 1 à 4, et Z représente un atome d'oxygène ou 2 atome d'hydrogène,
   et dans lesquels R₅ et R₆ sont tels que définis dans la formule (I),
les termes "alkyle inférieur", "alcoxy inférieur", "cycloalkyle", "acyle", et "aryle", étant tels que définis dans la formule (I),
caractérisé en ce que l'on fait réagir un composé de formule (I₈), 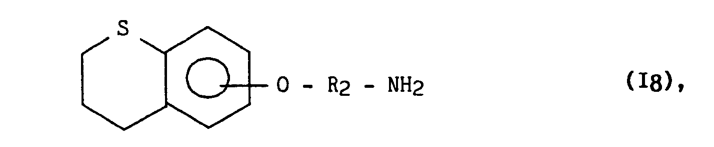
où R₂ est tel que défini précédemment,
avec un composé halogéné de formule (IIᵢ) :

X₅ - R₇ **(II**_{**i**}**),**

dans laquelle R₇ est tel que défini précédemment et X₅ représente un atome d'halogène,
pour donner suivant la stoechiométrie utilisée un composé de formule (I₇) ou (I_{7'}) tels que définis précédemment,
composés de formule (I₇) et (I_{7'}) qui peuvent être, si on le désire,
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- ou salifiés, par un acide ou une base pharmaceutiquement acceptable.

La présente invention concerne aussi le procédé de préparation des composés de formule (I₁) : 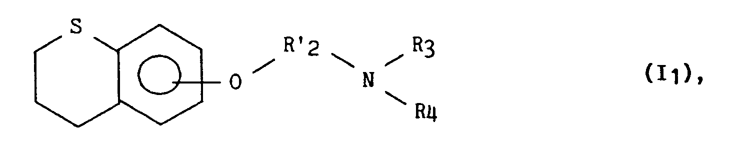
dans lequel R'₂, R₃ et R₄ sont tels que définis dans la formule (I), cas particulier des composés de formule (I), où R₁ représente un groupement de formule : 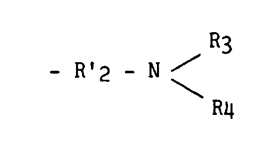
dans laquelle R'₂, R₃ et R₄ sont tels que définis précédemment,
caractérisé en ce que :
on fait réagir un thiochromanol de formule (II) : 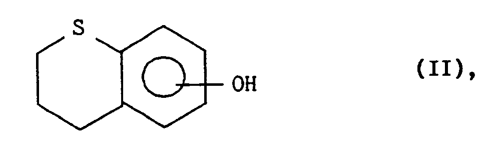
avec une halogénoalkylamine de formule (IIⱼ) : 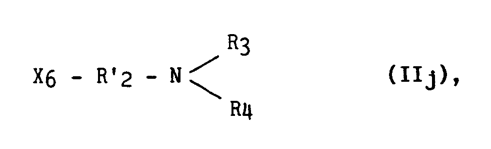
où R'₂, R₃ et R₄ sont tels que définis précédemment et où X₆ représente un atome d'halogène, pour donner un composé de formule (I₁) tel que défini précédemment,
composés de formule (I₁) qui peuvent être, si on le désire,
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- ou salifiés, par un acide ou une base pharmaceutiquement acceptable.

La présente invention s'étend au procédé de préparation des composés de formule (I₆), 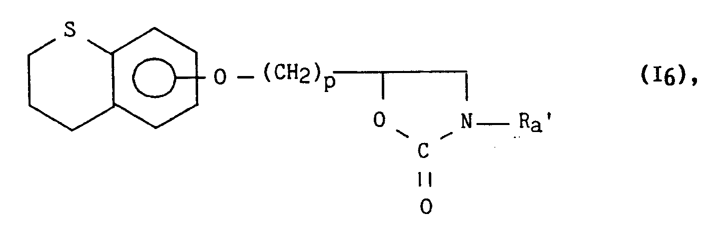
où p est tel que défini dans la formule (I) et Rₐ' représente un radical alkyle inférieur,
caractérisé en ce que l'on fait réagir un composé de formule (IIₖ) : 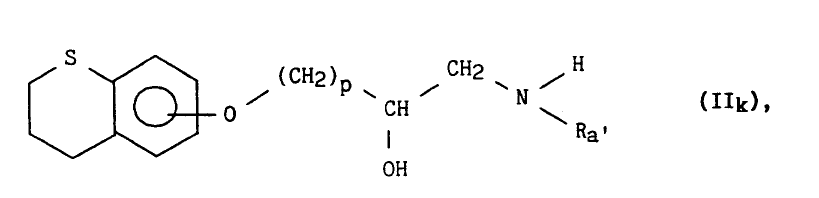
dans laquelle p et Ra' sont tels que définis précédemment, cas particulier des composés de formule (I) dans lesquels R₁ représente un groupement 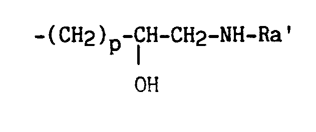
dans lequel Ra' et p sont tels
que définis précédemment, avec du N,N'-carbonyldiimidazole pour obtenir un composé de formule (I₆), tel que défini précédemment,
cas particulier des composés de formule (I), où R₁ représente un groupement de formule : 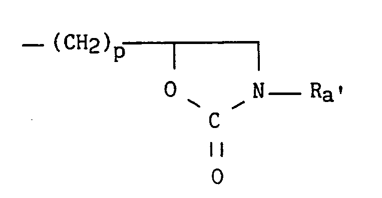
dans laquelle R_{a'} et p sont tels que définis précédemment,
composés de formule (I₆) qui peuvent être, si on le désire,
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs isomères optiques.

Les matières premières utilisées lors des procédés précédemment décrits sont soit comprises dans la littérature, soit aisément accessibles à l'homme de l'art.

La demanderesse a découvert que les composés de l'invention possédaient des propriétés pharmacologiques très intéressantes.

Les composés de l'invention présentent de façon surprenante une très forte affinité pour les récepteurs sérotoninergiques et certains composés de l'invention possèdent notamment un intense pouvoir antagoniste des récepteurs 5HT_{1A}.

Des essais de liaison aux récepteurs 5-HT_{1A} ont en effet montré que les composés de l'invention se comportent comme de très puissants ligands des récepteurs sérotoninergiques 5-HT_{1A}.

L'activité antagoniste des composés de l'invention a été mise en évidence in-vitro et se traduit in-vivo par une très forte activité anxiolytique (test dit des cages claires/obscures chez la souris, exemple F de la présente demande) associée à une remarquable activité antidépressive (test sur les échecs d' échappement chez le rat, exemple E de la présente demande).

De ce fait, les composés de formule générale (I) et leurs sels physiologiquement tolérables possèdent des propriétés pharmacologiques et thérapeutiques intéressantes, notamment des propriétés anxiolytiques et antidépressives.

Par ailleurs, les composés de l'invention présentent une bonne sélectivité vis-à-vis des récepteurs 5-HT_{1A}, par rapport aux récepteurs β₁ et β₂, ce qui est tout à fait surprenant au vue des composés les plus proches de l'état de l'art.

Cette sélectivité leur confère de remarquables propriétés anxiolytiques et antidépressives pures, dépourvues d'effets indésirables au niveau cardio-vasculaire.

Les composés de la présente invention peuvent ainsi être utilisés dans le traitement et la prévention du stress, de l'anxiété, de la dépression, des psychoses, de la schizophrénie, de la douleur et des troubles du comportement alimentaire et sexuel, de préférence dans le traitement de la dépression.

D'autre part, les composés de l'invention potentialisent de façon surprenante les effets des anti-dépresseurs connus et permettent leur efficacité immédiate (disparition de la latence d'une quinzaine de jours généralement observée).

La présente invention a également pour objet une composition pharmaceutique contenant comme principe actif un composé de formule (I) ou un de ses sels d'addition à un acide ou à une base pharmaceutiquement acceptable, en combinaison avec un ou plusieurs excipients ou véhicules pharmaceutiquement acceptables.

La présente invention a également pour objet une composition pharmaceutique contenant comme principe actif au moins un composé selon la formule (I), ou un de ses sels d'addition à un acide ou à une base pharmaceutiquement acceptable, associé à au moins un principe actif antidépresseur connu, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, rectale, nasale ou parentérale et notamment les comprimés, les dragées, les gélules, les paquets, les sachets, les granules, les pilules, les granulés, les suppositoires, les crèmes, les pommades, les aérosols, les capsules, les gels dermiques et les solutions injectables ou buvables

La posologie varie d'un individu à l'autre, selon l'âge, le poids, et le sexe du malade, la voie d'administration choisie, la nature et l'intensité de l'affection. Les doses utilisées s'échelonnent entre 0,1 et 100 mg pour un traitement (particulièrement entre 0,1 et 10 mg, par exemple entre 1 et 10 mg), divisibles en 1 à 3 prises par 24 h.

Les exemples suivants illustrent l'invention et ne la limitent en aucune façon.

### EXEMPLE 1 : 8-{{3-[4-(2-METHOXYPHENYL)PIPERAZIN-1-YL]-2-HYDROXYPROPYL} OXY}THIOCHROMANE

### STADE A : 8-(2,3-EPOXYPROPANOXY)THIOCHROMANE

On dissout sous argon 5 g (30,08 mmoles) de 8-thiochromanol dans 30 cm³ de N,N-diméthylformamide. On ajoute 800 mg (33,09 mmoles) d'hydrure de sodium et on porte à 60° C sous agitation.

Après 15 mn, on ajoute 18,8 cm³ (240,61 mmoles) d'épichlorhydrine. On laisse 1 heure à 60° C.

On évapore le N,N-diméthylformamide. On reprend lentement le résidu par de l'eau et on l'extrait au dichlorométhane. On purifie sur colonne de silice pour obtenir le 8-(2,3-époxypropanoxy)thiochromane (éluant : dichlorométhane pur).
On récupère le 8-(2,3-époxypropanoxy)thiochromane sous forme d'une huile incolore.
Rendement : 65 %

### STADE B : 8-{{3-[4-(2-METHOXYPHENYL)PIPERAZIN-1-YL]-2-HYDROXY PROPYL}OXY}THIOCHROMANE

On dissout 140 mg (0,63 mmoles) du composé obtenu au stade A dans 1,5 cm³ de tétrahydrofurane anhydre. On ajoute 600 mg (3,15 mmoles) de 1-(2-méthoxyphényl)pipérazine, diluée dans 1,5 cm³ de tétrahydrofurane anhydre.

On porte au reflux sous argon et sous agitation pendant 15 heures. On évapore le tétrahydrofurane, on reprend le résidu par de l'eau, on l'extrait par du dichlorométhane et on le sèche sur du sulfate de magnésium.

On purifie le produit sur une colonne de silice (éluant : méthanol/dichlorométhane ; 5 : 95).

On récupère 255 mg de 8-{{3-[4-(2-méthoxyphényl)pipérazin-1-yl] 2-hydroxy propyl}oxy}thiochromane, que l'on lave à l'éther.
Rendement : 98 %
Point de fusion : 134-135° C

| Résonance magnétique nucléaire (CDCl₃ + D₂O) : | |
|---|---|
| CH₂ - CH₂ - S | 2,01 à 2,11 ppm (2H, m) |
| CH₂ - N | 2,69 à 2,72 ppm (4H, m) |
| CH₂ - N ; CH₂ - aromatique | 2,83 à 2,92 ppm (4H, m) |
| CH₂ - S (J = 5,8H_{z}) | 3,00 ppm (2H, t) |
| CH₂ - N - aromatique | 3,13 à 3,17 ppm (4H, m) |
| CH₃ - O | 3,87 ppm (3H, s) |
| CH₂ - O | 4,09 ppm (2H, m) |
| CH - OH | 4,19 ppm (1H, m) |
| aromatique (J = 7,9 H_{z}) | 6,72 ppm (2H, d) |
| aromatique | 6,84 à 7,06 ppm (5H, m) |

### EXEMPLE 2 : 8-{{4-[4-(2-METHOXYPHENYL)PIPERAZIN-1-YL]BUTYL}OXY} THIOCHROMANE

### STADE A : 8-[(4-BROMOBUTYL)OXY]THIOCHROMANE

On dissout 300 mg de 8-thiochromanol dans 3 cm³ de N,N-diméthylformamide sous argon. On ajoute 430 mg (1,985 mmoles) de 1,4-dibromobutane dilués dans 3 cm³ de N,N-diméthylformamide.

On ajoute ensuite 750 mg de carbonate de potassium à la spatule.

On porte à 65° C sous argon et sous agitation pendant 3h et 30 mn.

On évapore le N,N-diméthylformamide. on reprend le résidu par de l'eau et on l'extrait au dichlorométhane, avant de le purifier sur une colonne de silice (éluant : éther / éther de pétrole ; 15 : 85). On récupère 240 mg de cristaux blancs de 8-[(4-bromobutyl)oxy] thiochromane.

### STADE B : 8-{{4-[4-(2-METHOXYPHENYL)PIPERAZIN-1-YL]BUTYL}OXY} THIOCHROMANE

On dissout 1,5 g (4,98 mmoles) du composé obtenu au stade A dans 15 cm³ d'acétonitrile sous argon. On ajoute 0,94 g (7,47 mmoles) de N,N-diisopropyléthylamine, diluée dans 11 cm³ d'acétonitrile puis 1,44 g (0,747 mmoles) de 1-(2-méthoxyphényl)pipérazine dissoute dans 11 cm³ d'acétonitrile.

On porte au reflux pendant 15 heures, puis on évapore l'acétonitrile, on reprend le résidu par de l'eau, on l'extrait au dichlorométhane et on le sèche sur sulfate de magnésium avant de le purifier sur colonne de silice (éluant : méthanol / dichlorométhane ; 5 : 95).

On récupère 1,98 g de 8-{{4-[4-(2-méthoxyphényl)pipérazin-1-yl]butyl}oxy}thiochromane.
Rendement : 97 %
Point de fusion : 61° C

| Résonance magnétique nucléaire (CDCl₃ + D₂O) : | |
|---|---|
| CH₂ - CH₂ - N | 1,75 à 1,77 ppm (2H, m) |
| CH₂ - CH₂ - O | 1,87 à 1,89 ppm (2H, m) |
| CH₂ - CH₂ - S | 2,00 à 2,10 ppm (2H, m) |
| CH₂ - N (J = 7,2H_{z}) | 2,50 ppm (2H, t) |
| CH₂ - N (J = 5,5H_{z}) | 2,68 ppm (4H, t) |
| CH₂ - Ar (J = 6,2H_{z}) | 2,83 ppm (2H, t) |
| CH₂ - S (J = 5,8H_{z}) | 3,00 ppm (2H, t) |
| CH₂ - N - Ar (J = 5,5H_{z}) | 3,10 ppm (4H, t) |
| CH₃ - O | 3,89 ppm (3H, s) |
| CH₂ - O (J = 6,3H_{z}) | 4,03 ppm (2H, t) |
| aromatique | 6,65 à 7,05 ppm (7H, m) |

### EXEMPLE 3 : 8-{{4-[4-(3-TRIFLUOROMETHYLPHENYL)PIPERAZIN-1-YL]BUTYL} OXY}THIOCHROMANE

En procédant comme dans l'exemple 2 mais en remplaçant dans le stade B la 1-(2-méthoxyphényl)pipérazine par la 1-(3-trifluorométhylphényl)pipérazine, on obtient le composé du titre.
Rendement : 90 %
Point de fusion (oxalate) : 105° C

| Résonance magnétique nucléaire (CDCl₃ + D₂O) : | |
|---|---|
| CH₂ - CH₂ - O, CH₂ - CH₂ - N | 1,72 à 1,90 ppm (4H, m) |
| CH₂ - CH₂ - S | 2,00 à 2,10 ppm (2H, m) |
| CH₂ - N (J = 7H_{z}) | 2,50 (2H, t) |
| CH₂ - N (J = 5,5H_{z}) | 2,65 (4H, t) |
| CH₂ - Ar (J = 6,2H_{z}) | 2,81 (2H, t) |
| CH₂ - S (J = 5,8H_{z}) | 3,00 (2H, t) |
| CH₂ - N - Ar (J = 5,5H_{z}) | 3,24 (4H, t) |
| CH₂ - O (J = 6,3H_{z}) | 4,05 (2H, t) |
| aromatique (J = 7,9H_{z}) | 6,65 ppm (2H, t) |
| aromatique (J = 7,9H_{z}) | 6,93 ppm (1H, t) |
| aromatique | 7 à 7,12 ppm (3H, m) |
| aromatique (J = 8,3H_{z}) | 7,32 ppm (1H, t) |

### EXEMPLES 4 ET 5 :

En procédant comme dans les exemples 2 et 3 mais en remplaçant le 8-thiochromanol par le 7-thiochromanol, on obtient les composés suivants :

### EXEMPLE 4 : 7-{{4-[4-(2-METHOXYPHENYL)PIPERAZIN-1-YL]BUTYL}OXY} THIOCHROMANE

### EXEMPLE 5 : 7-{{4-[4-(3-TRIFLUOROMETHYLPHENYL)PIPERAZIN-1-YL]BUTYL}OXY} THIOCHROMANE

### EXEMPLE 6 : 8-{[4-BIS(4-FLUOROPHENYL)METHYLPIPERAZIN-1-YL]BUTYL]OXY} THIOCHROMANE

En procédant comme dans l'exemple 2 mais en remplaçant dans le stade B la 1-(2-méthoxyphényl)pipérazine par la 1-bis(4-fluorophényl)méthyl pipérazine, on obtient le composé du titre.
Rendement : 96 %
Point de fusion (oxalate) : 194°C

| Résonance magnétique nucléaire (CDCl₃) : | |
|---|---|
| CH₂ - CH₂ - N | 1,69 à 1,72 ppm (2H, m) |
| CH₂ - CH₂ - O | 1,80 à 1,84 ppm (2H, m) |
| CH₂ - CH₂ - S | 2,00 à 2,10 ppm (2H, m) |
| CH₂ - N | 2,29 à 2,58 ppm (10H, m) |
| CH₂ - aromatique (J = 6,2H_{z}) | 2,81 ppm (2H, t) |
| CH₂ - S (J = 5,8H_{z}) | 2,98 ppm (2H, t) |
| CH₂ - O (J = 6,3H_{z}) | 4,00 ppm (2H, t) |
| CH - aromatique | 4,21 ppm (1H, s) |
| aromatique (J = 7,9H_{z}) | 6,64 ppm (2H, t) |
| aromatique (J = 7,9H_{z}) | 6,91 ppm (1H, t) |
| aromatique (J = 8,3H_{z}) | 6,96 ppm (4H, t) |
| aromatique (J = 8,3H_{z}) | 7,34 et 7,36 ppm (4H, 2d) |

### EXEMPLE 7 : 8-{[3-(MORPHOLIN-4-YL)2-HYDROXYPROPYL]OXY}THIOCHROMANE

En procédant comme dans l'exemple 1 mais en remplaçant dans le stade B la 1-(2-méthoxyphényl)pipérazine par la morpholine, on obtient le composé du titre.

### EXEMPLE 8 : 8-[6-PERHYDROAZEPINYLHEXYL)OXY]THIOCHROMANE

En procédant comme dans l'exemple 1 mais en remplaçant dans le stade B la 1-(2-méthoxyphényl)pipérazine par la perhydroazépine, on obtient le composé du titre.

### EXEMPLE 9 : 8-{[4-(7,9-DIOXO-8-AZASPIRO[4,5]DECAN-8-YL)BUTYL]OXY} THIOCHROMANE

A 1g (6,015 mmoles) de 8-thiochromanol en solution dans 10 cm³ de N, N-diméthylformamide, sont ajoutés sous agitation, 2 g (6,62 mmoles) de 8-(4-bromobutyl)-8-azaspiro[4,5]décane 7,9-dione, 2,5 g (18,04 mmoles) de carbonate de potassium et une quantité catalytique d'iodure de potassium.

Le mélange est porté à 60° C sous agitation pendant 20 heures, puis refroidi et le solvant est évaporé sous pression réduite. Le résidu est repris par 10 cm3 d'eau et le produit est extrait au dichlorométhane.

Après sèchage sur sulfate de magnésium, et évaporation du solvant, on obtient 2,29 g de 8-[(4-(7,9-dioxo-8-azaspiro[4,5]décan-8-yl)butyl)oxy]thiochromane, après purification par chromatographie sur colonne de silice (éluant : diéthyl éther / éther de pétrole ; 1 : 1)
Rendement : 98 %
Point de fusion : 69°C

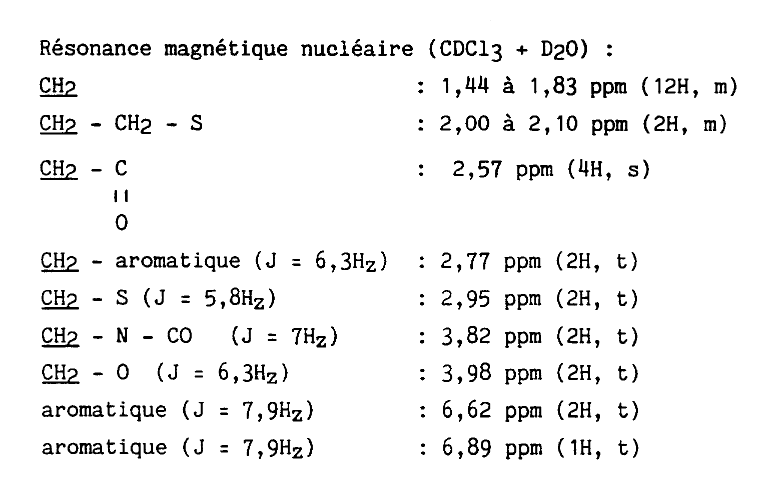

### EXEMPLES 10 A 22 :

En procédant comme dans l'exemple 9 mais en remplaçant la 8-(4-bromobutyl)-8-azaspiro[4,5]décane 7,9-dione par les composés halogénés appropriés, on obtient de la même façon :

### EXEMPLE 10 : 8-{[4-(4-HYDROXYPIPERIDIN-1-YL)BUTYL]OXY}THIOCHROMANE

### EXEMPLE 11 : 8-{{4-[4-(ETHOXYCARBONYL)PIPERIDIN-1-YL]BUTYL}OXY} THIOCHROMANE

### EXEMPLE 12 : 8-{[2-(DIETHYLAMINO)ETHYL]OXY}THIOCHROMANE

### EXEMPLE 13 : 8-{[(TERTIOBUTYLAMINO)ETHYL]OXY}THIOCHROMANE

### EXEMPLE 14 : 8-{[3-(CYCLOHEXYLAMINO)PROPYL]OXY}THIOCHROMANE

### EXEMPLE 15 : 8-{[4-(N-ETHYL N-CYCLOPENTYLAMINO)BUTYL]OXY}THIOCHROMANE

### EXEMPLE 16 : 8-{[2-(7,9-DIOXO-8-AZASPIRO[4,5]DECAN-8-YL)ETHYL]OXY} THIOCHROMANE

### EXEMPLE 17 : 8-{{2-{[4-(7,9-DIOXO-8-AZASPIRO[4,5]DECAN-8-YL)BUTYL] AMINO}ETHYL}OXY}THIOCHROMANE

### EXEMPLE 18 : 8-{{3-[4-(2-METHOXYPHENYL)PIPERAZIN-1-YL]PROPYL}OXY} THIOCHROMANE

### EXEMPLE 19 : 8-{[4-(1-AZASPIRO[5,5]UNDECAN-1-YL)BUTYL]OXY}THIOCHROMANE

### EXEMPLE 20 : 8-{{2-{4-[2-(METHOXY)PHENYL]PIPERIDIN-1-YL}ETHYL}OXY} THIOCHROMANE

### EXEMPLE 21 : 8-{{2-{[2-(PHTALIMIDO)ETHYL]AMINO}ETHYL}OXY}THIOCHROMANE

### EXEMPLE 22 : 8-{{2-{[2-(PHTALIMIDOCARBONYL)ETHYL]AMINO}ETHYL} OXY} THIOCHROMANE

### EXEMPLE 23 : 8-{[3-(3-METHYLCYCLOHEXYLAMINO)2-HYDROXYPROPYL]OXY} THIOCHROMANE

En procédant comme dans l'exemple 1 mais en remplaçant dans le stade B la 1-(2-méthoxyphényl)pipérazine par la 3-méthylcyclohexylamine, on obtient le composé du titre.

### EXEMPLE 24 : 8-{{3-[4-(PYRIMIDIN-2-YL)PIPERAZIN-1-YL]2-HYDROXYPROPYL} OXY}THIOCHROMANE

En procédant comme dans l'exemple 1 mais en remplaçant dans le stade B la 1-(2-méthoxyphényl)pipérazine par la 1-(pyrimidin-2-yl)pipérazine, on obtient le composé du titre.
Rendement : 88 %
Point de fusion (oxalate) : 200° C

| Résonance magnétique nucléaire (CDCl₃ + D₂O) : | |
|---|---|
| CH₂ - CH₂ - S | 2,00 à 2,10 ppm (2H, m) |
| CH₂ - N | 2,50 à 2,77 ppm (6H, m) |
| CH₂ - aromatique (J = 6,3H_{z}) | 2,81 ppm (2H, t) |
| CH₂ - S (J = 5,8H_{z}) | 3,00 ppm (2H, t) |
| CH₂ - N (J = 6,2H_{z}) | 3,88 ppm (4H, t) |
| CH₂ - O (J = 6,5H_{z}) | 4,08 ppm (2H, d) |
| CH - OH | 4,16 à 4,20 ppm (1H, m) |
| aromatique (J = 5H_{z}) | 6,50 ppm (1H, t) |
| aromatique (J = 7,9H_{z}) | 6,71 ppm (2H, d) |
| aromatique (J = 7,9H_{z}) | 6,95 ppm (1H, t) |
| aromatique (J = 5H_{z}) | 8,31 ppm (2H, d) |

### EXEMPLE 25 : 8-{{3-[4-(3-TRIFLUOROMETHYLPHENYL)PIPERAZIN-1-YL] 2-HYDROXYPROPYL}OXY}THIOCHROMANE

En procédant comme dans l'exemple 1 mais en remplaçant dans le stade B la 1-(2-méthoxyphényl)pipérazine par la 1-(3-trifluorométhylphényl)pipérazine, on obtient le composé du titre.
Rendement : 90 %
Point de fusion (oxalate) : 226° C

| Résonance magnétique nucléaire (CDCl₃ + D₂O) : | |
|---|---|
| CH₂ - CH₂ - S | 2,00 à 2,10 ppm (2H, m) |
| CH₂ - N | 2,67 à 2,70 ppm (4H, m) |
| CH₂ - aromatique, CH₂ - N | 2,81 à 2,84 ppm (4H, m) |
| CH₂ - S (J = 5,9H_{z}) | 3,00 ppm (2H, t) |
| CH₂ - N - aromatique (J = 5,1H_{z}) | 3,27 ppm (4H, t) |
| CH₂ - O | 4,04 à 4,06 ppm (2H, m) |
| CH - OH | 4,17 ppm (1H, m) |
| aromatique (J = 7,9H_{z}) | 6,69 ppm (2H, d) |
| aromatique (J = 7,9H_{z}) | 6,94 ppm (1H, t) |
| aromatique | 7,06 à 7,08 ppm (3H, m) |
| aromatique (J = 7,9H_{z}) | 7,35 ppm (1H, t) |

### EXEMPLES 26 ET 27 :

En procédant comme dans l'exemple 1 mais en remplaçant dans le stade B la 1-(2-méthoxyphényl)pipérazine par les amines appropriées, on obtient les composés suivants :

### EXEMPLE 26 : 8-{[3-(4-BENZYLPIPERAZIN-1-YL) 2-HYDROXYPROPYL]OXY} THIOCHROMANE

### EXEMPLE 27 : 8-{{3-{4-[2-(2-METHYLPHENYL)ETHYL]PIPERAZIN-1-YL} 2-HYDROXYPROPYL}OXY}THIOCHROMANE

### EXEMPLE 28 : 8-{[3-(7,9-DIOXO-8-AZASPIRO[4,5]DECAN-8-YL) 2-HYDROXYPROPYL] OXY}THIOCHROMANE

En procédant comme dans l'exemple 1 mais en remplaçant dans le stade B la 1-(2-méthoxyphényl)pipérazine par la 8-azaspiro[4,5]décane 7,9-dione, on obtient le composé du titre (huile).

| Résonance magnétique nucléaire (CDCl₃ + D₂O) : | |
|---|---|
| CH₂ | 1,51 à 1,55 ppm (4H, m) |
| CH₂ | 1,68 à 1,72 ppm (4H, m) |
| CH₂ - CH₂ - S | 2,00 à 2,12 ppm (2H, m) |
| CH₂ - CO | 2,65 ppm (4H, s) |
| CH₂ - aromatique (J = 6,2H_{z}) | 2,81 ppm (2H, t) |
| CH₂ - S (J = 5,8H_{z}) | 3,00 ppm (2H, t) |
| CH₂ - N - CO, CH - O-aromatique | 3,96 à 4,10 ppm (3H, m) |
| CH - OH | 4,13 à 4,17 ppm (1H, m) |
| CH - O - aromatique (J = 8,5H_{z}) | 4,29 ppm (1H, dd) |
| aromatique (J = 7,9H_{z}) | 6,65 et 6,69 ppm (2H, 2d) |
| aromatique (J = 7,9H_{z}) | 6,92 ppm (1H, t) |

### EXEMPLE 29 : 8-{[3-(4-AZASPIRO[5,5]UNDECAN-4-YL) 2-HYDROXYPROPYL]OXY} THIOCHROMANE

En procédant comme dans l'exemple 1 mais en remplaçant dans le stade B la 1-(2-méthoxyphényl)pipérazine par le 1-azaspiro[5,5]undécane,on obtient le composé du titre.

### EXEMPLE 30 A 32 :

En procédant comme dans les exemples 1, 25 et 28 mais en remplaçant le 8-thiochromanol par du 6-thiochromanol, on obtient les composés suivants :

### EXEMPLE 30 : 6-{{3-[4-(2-METHOXYPHENYL)PIPERAZIN-1-YL] 2-HYDROXYPROPYL} OXY}THIOCHROMANE

### EXEMPLE 31 : 6-{{3-[4-(3-TRIFLUOROMETHYLPHENYL)PIPERAZIN-1-YL] 2-HYDROXYPROPYL}OXY}THIOCHROMANE

### EXEMPLE 32 : 6-{[3-(7,9-DIOXO-8-AZASPIRO[4,5]DECAN-8-YL)2-HYDROXYPROPYL}OXY}THIOCHROMANE

### EXEMPLE 33 : 8-{[5-(7,9-DIOXO-8-AZASPIRO[4,5]DECAN-8-YL) 4-HYDROXYPENTYL] OXY}THIOCHROMANE

### EXEMPLE 34 : 8-{{3-{N,N-DI[4-(7,9-DIOXO-8-AZASPIRO[4,5]DECAN-8-YL)BUT-1-YL]AMINO]-2-HYDROXYPROPYL}OXY}THIOCHROMANE

### STADE A : 8-(2,3-EPOXYPROPANOXY)THIOCHROMANE

On opère comme dans le stade A de l'exemple 1, en partant de 1,72 g (10,34 mmoles) de thiochromanol et de 7,653 g (82,72 mmoles) d'épichlorhydrine. On obtient 1,5 g (6,721 mmoles) de 2,3-époxy propanoxy thiochromane.
Rendement : 65 %

### STADE B : 8-[(3-AMINO 2-HYDROXYPROPYL)OXY]THIOCHROMANE

On fait réagir 1,494 g (6,72 mmoles) du composé obtenu au stade A, en solution dans un mélange dioxane-eau, avec 0,612 g (9,41 mmoles) d'azidure de sodium. Le mélange est chauffé à reflux pendant 8 heures puis refroidi, et le solvant est évaporé sous pression réduite.

Le brut réactionnel est repris dans de l'eau puis extrait au dichlorométhane.

La phase organique est séchée sur sulfate de magnésium puis évaporée, et on obtient, après purification sur colonne de silice (éluant : éther de pétrole - diéthyléther), 1,23 g d'azoture de 2-hydroxy 3-[(thiochromane-8-yl)oxy]propane, (rendement : 69 %), qui est placé, en présence d'éthanol et de catalyseur de Lindlar dans un hydrogénateur à 25° C pendant 8 heures pour donner du 8-[(3-amino 2-hydroxypropyl) oxy]thiochromane.
Rendement : 97 %

### STADE C : 8-{{3-[N,N-DI(4-(7,9-DIOXO-8-AZASPIRO[4,5]DECAN-8-YL)BUT-1-YL)AMINO]-2-HYDROXYPROPYL]OXY]THIOCHROMANE

On dissout 1,36 g (4,498 mmoles) de 8-(4-bromobutyl)-8-azaspiro[4,5] décane 7,9-dione dans 11 cm³ d'acétonitrile sous argon. On ajoute 1,077 g (4,498 mmoles) de composé obtenu au stade B puis 1,21 g de diisopropyléthylamine dilués dans 14,5 cm³ d'acétonitrile.

On porte au reflux sous argon et sous agitation pendant 15 heures.

On évapore l'acétonitrile, on reprend le résidu dans de l'eau avant de l'extraire au dichlorométhane. Après séchage sur sulfate de magnésium on purifie alors le 8-{(3-[N,N-di(4-(7,9-dioxo-8-azaspiro[4,5]décan-8-yl)but-1-yl)amino] 2-hydroxypropyl)oxy}thiochromane obtenu sur une colonne de silice (éluant : méthanol-dichlorométhane ; 5 : 95)
Point de fusion (oxalate) : 72-73° C

### EXEMPLE 35 : 8-{{3-[N,N-DI(2-PHENYLETHYL)AMINO] 2-HYDROXYPROPYL} OXY}THIOCHROMANE

En procédant comme dans l'exemple 34 mais en remplaçant dans le stade C la 8-(4-bromobutyl)-8-azaspiro[4,5]décane 7,9-dione par le 1-bromo 2-phényléthyl, on obtient le composé de titre.

### EXEMPLE 36 : 8-{{[3-(N-TERTIOBUTYL N-BENZYLAMINO) 2-HYDROXY]PROPYL}OXY} THIOCHROMANE

En procédant comme dans l'exemple 1 mais en remplaçant dans le stade B la 1-(2-méthoxyphényl)pipérazine par la N-tertiobutylbenzylamine, on obtient le composé du titre.

### EXEMPLE 37 : 8-{{3-[N-SEC-BUTYL N-(2,6-DIHYDROXYBENZYL)AMINO] 2-HYDROXY)PROPYL]OXY}THIOCHROMANE

En procédant comme dans l'exemple 1 mais en remplaçant dans le stade B la 1-(2-méthoxyphényl)pipérazine par la N-sec-butyl 2,6-dihydroxybenzylamine, on obtient le composé du titre.

### EXEMPLE 38 : 8-{{3-[4-(2-METHOXYPHENYL)PIPERAZIN-1-YL]2-METHOXYPROPYL}OXY}THIOCHROMANE

On dissout sous argon et sous agitation 100 mg (2,41.10⁻⁴ mol) du composé de l'exemple 1 dans 2 cm³ de tétrahydrofurane anhydre.

On ajoute 9 mg (3,62.10⁻⁴ mol) d'hydrure de sodium par petites portions. On ajoute ensuite 171 mg (1,20 mmol) de iodure de méthyle goutte à goutte.

On laisse 30 minutes à température ambiante. On évapore le solvant puis on reprend par de l'eau. On extrait au dichlorométhane. On sèche sur du sulfate de magnésium.

On purifie sur une colonne de silice (éluant : éther / éther de pétrole ; 1 : 1 puis éther 100 %).

On recueille 90 mg de 8-{{3-[4-(2-méthoxyphényl)pipérazin-1-yl]2-méthoxypropyl}oxy}thiochromane.
Rendement : 87 %
Point de fusion : 123°C

### EXEMPLE 39 : 8-[(3-TERTIOBUTYLAMINO 2-METHOXY PROP-1-YL)OXY]THIOCHROMANE

En procédant comme dans l'exemple 1 mais en remplaçant dans le stade B la 1-(2-méthoxyphényl)pipérazine par la N-tertiobutylamine, puis en procédant, avec le composé obtenu, comme pour l'exemple 38, on obtient le produit du titre.
Rendement : 83 %
Point de fusion (oxalate) : 185° C

| Résonance magnétique nucléaire (CDCl₃) : | |
|---|---|
| CH₃ - C | 1,13 ppm (9H, s) |
| NH | 1,66 ppm (1H, massif) |
| CH₂ - CH₂ - S | 2,00 à 2,10 ppm (2H, m) |
| CH₂ - aromatique, CH₂ - N | 2,78 à 2,82 ppm (4H, m) |
| CH₂ - S (J = 5,8H_{z}) | 3,00 ppm (2H, t) |
| CH₃ - O | 3,54 ppm (3H, s) |
| CH - O | 3,71 à 3,75 ppm (1H, m) |
| CH₂ - O | 4,06 à 4,10 ppm (2H, m) |
| aromatique (J = 7,9H_{z}) | 6,66 ppm (2H, d) |
| aromatique (J = 7,9H_{z}) | 6,94 ppm (1H, t) |

### EXEMPLE 40 : 8-{{3-[4-(3-TRIFLUOROMETHYLPHENYL)PIPERAZIN-1-YL]2-METHOXYPROPYL}OXY]THIOCHROMANE

En procédant comme pour l'exemple 38, mais en partant du composé de l'exemple 25, on obtient le composé du titre.

### EXEMPLE 41 : 8-{{3-[4-(2-METHOXYPHENYL)PIPERAZIN-1-YL]2-(ACETYL-OXY) PROPYL}OXY}THIOCHROMANE

De façon analogue à l'exemple 38, mais en utilisant le chlorure d'acétyle à la place de l'iodure de méthyle, on obtient le 8-{{3-[4-(2-méthoxyphényl)pipérazin-1-yl] 2-(acétyloxy) propyl} oxy}thiochromane.

### EXEMPLE 42 : 8-{[3-(7,9-DIOXO-8-AZASPIRO[4,5]DECAN-8-YL)PROPYL]OXY} THIOCHROMANE

En procédant comme dans l'exemple 2 mais en remplaçant dans le stade A le 1,4-dibromobutane par le 1,3-dibromopropane et en remplaçant dans le stade B la 1-(2-méthoxyphényl)pipérazine par l'amine appropriée, on obtient le composé du titre.
Point de fusion : inférieur à 50°C

### EXEMPLE 43 : 8-{{3-[4-(2-METHOXYPHENYL)PIPERAZIN-1-YL]PROPYL}OXY} THIOCHROMANE

En procédant comme dans l'exemple 2 mais en remplaçant au stade A le 1,4-dibromobutane par le 1,3-dibromopropane, on obtient le composé du titre.
Point de fusion (oxalate) : 115° C

### EXEMPLE 44 : 8-{{2-[4-(2-METHOXYPHENYL)PIPERAZIN-1-YL]ETHYL}OXY} THIOCHROMANE

### STADE A : 8-[(2-BROMOETHYL)OXY]THIOCHROMANE

Dissoudre 1 g (6,015 mmol) de 8-thiochromanol dans 2,26 g (12,03 mmol) de 1,2-dibromoéthane. Ajouter goutte à goutte 5,6 cm³ d'une solution de NaOH 1,6 N. Porter à 100°C, après 2h, la solution atteint un pH de 7. Laisser refroidir. Ajouter 100 cm³ d'une solution de NaOH 2N. Extraire au CH₂Cl₂ puis sécher sur MgSO₄. Après purification sur colonne de silice (éluant : Ether de pétrole/CH₂Cl₂ : 2-1). On obtient le composé attendu.
Rendement : 32 %
Point de fusion : 64° C

### STADE B : 8-{{2-[4-(2-METHOXYPHENYL)PIPERAZIN-1-YL]ETHYL}OXY} THIOCHROMANE

Sous argon et sous agitation, dissoudre 710 mg (2,6 mmol) de dérivé bromé dans 5 cm³ de CH₃CN. Ajouter la N,N diisopropyléthylamine dissoute dans 6 cm³ de CH₃ CN puis la 1-(2-méthoxyphényl)-pipérazine dissoute dans 6 cm³ de CH₃ CN. Porter à reflux une nuit. Evaporer le solvant. Reprendre par H₂O puis extraire au CH₂Cl₂ et sécher sur MgSO₄. Purifier sur colonne de silice (éluant : éther).
Rendement : 75 %
Point de fusion : 79° C

### EXEMPLE 45 : 8-{[(2-OXO 3-TERTIOBUTYL OXAZOLIDIN-5-YL)METHYL]OXY} THIOCHROMANE

A 0,2 g (0,677 mmoles) de 8-{[3(tertiobutylamino) 2-hydroxypropyl] oxy}thiochromane en solution dans 10 cm³ de tétrahydrofurane anhydre, on ajoute 1,63 g (10,08 mmoles) de N,N'-carbonyldiimidazole.

Le mélange est chauffé à reflux sous agitation pendant 2 heures, puis refroidi et le solvant est évaporé sous pression réduite.

Le brut réactionnel est ensuite purifié par chromatographie sur colonne de silice (solvant : dichloro méthane).

On obtient 0,21 g de 8-{[(2-oxo 3-tertiobutyl oxazolidin-5-yl) méthy]oxy}thiochromane.
Rendement : 95 %
Point de fusion : 90° C

### EXEMPLE 46 : 8-{[(2-OXO OXAZOLIDINE-5-YL)METHYL]OXY}THIOCHROMANE

A 1,7 g (7,1 mmoles) de 8-[(3-amino-2-hydroxypropyl)oxy]thiochromane obtenu au stade B de l'exemple 34, en solution dans 10 cm³ de tétrahydrofurane anhydre, on ajoute 17,22 g de N,N'-carbonyldiimidazole.

Le mélange est chauffé à reflux sous agitation pendant 2 heures, puis refroidi et le solvant est évaporé sous pression réduite.

Le brut réactionnel est ensuite purifié par chromatographie sur colonne de silice (éluant : dichlorométhane).

On obtient 0,86 g de 8-{[(2-oxo-oxazolidine-5-yl) méthyl]oxy} thiochromane.
Rendement : 51 %
Point de fusion : 152-153° C

### EXEMPLE 47 : 8-{{4-OXO-4-[4-(2-METHOXYPHENYL)PIPERAZIN-1-YL]BUTYL}OXY} THIOCHROMANE

### STADE A : 4-[(THIOCHROMANE-8-YL)OXY]BUTYRATE D'ETHYLE

Sous argon et sous agitation, dissoudre 1,5 g (9,02 mmol) de 8-thiochromanol dans 5 cm³ de diméthylformamide (DMF). Ajouter 3,74 g (27,06 mmol) de K₂ CO₃ puis, goutte à goutte, additionner, 1,94 g (9,92 mmol) de 4-bromobutyrate d'éthyle dissous dans 6 cm³ de DMF. Porter à 60°C pendant 5 h. Evaporer la DMF. Reprendre par H₂O. Extraire au CH₂Cl₂ puis sécher sur MgSO₄. Après purification sur colonne de silice (éluant : Ether/éther de pétrole : 3-7) on récupère 2,48 g du composé du titre (solide blanc).
Rendement : 98 %
Point de fusion : 31° C

### STADE B : ACIDE-4-[(THIOCHROMAN-8-YL)OXY]BUTYRIQUE

Sous argon et sous agitation, dissoudre 2,25 g (8,02 mmol) du composé obtenu au stade A dans 30 cm³ de méthanol. Ajouter goutte à goutte 4,5 cm³ d'une solution d'hydroxyde de potassium à 10 %. Après 2 h à température ambiante, évaporer le méthanol. Acidifier très lentement avec une solution d'acide chlorhydrique 2 N. Extraire le précipité formé au CH₂Cl₂. Sécher sur MgSO₄.
On récupère 1,94 g d'un solide blanc.
Rendement : 96 %
Point de fusion : 115°C

### STADE C : 8-{{4-OXO-4-[4-(2-METHOXYPHENYL)PIPERAZIN-1-YL]BUTYL}OXY} THIOCHROMANE

Sous argon et sous agitation, dissoudre 700 mg (2,77 mmol) de l'acide obtenu au stade précédent dans 5 cm³ de DMF. Refroidir dans la glace. Ajouter 590 mg (3,05 mmol) de 1-(3-diméthylaminopropyl)-3-éthylcarbodiimide et 425 mg (2,77 mmol) de 1-hydroxybenzotriazole hydrate puis 590 mg (3,05 mmol) de 1-(2-méthoxyphényl)-pipérazine dissous dans 5 cm³ de DMF. Laisser revenir à température ambiante. Après 24h00, évaporer la DMF. Reprendre par H₂O puis extraire au CH₂Cl₂. Sécher sur Mg SO₄. Purifier sur colonne de silice (éluant : éther). On récupère 1,12 g d'une huile jaune (base).
Rendement : 95 %
Point de fusion (fumarate) : 235° C

### EXEMPLE 48 : 8-{{3-[4-(2-METHOXYPHENYL)PIPERAZIN-1-YL]-3-OXOPROPYL}OXY} THIOCHROMANE

Sous argon, dissoudre 110 mg (4,61.10⁻⁴ mol) d'acide 3-[(thiochroman-8-yl)oxy]propionique (Indian Journal of Chemistry (1977) 15 pp 715-719), dans 1,5 cm³ de diméthylformamide. Refroidir le ballon dans un bain de glace. Ajouter 100 mg (5,07.10⁻⁴ mol) de chlorhydrate de 1-(3-diméthylaminopropyl)-3-éthylcarbodiimide, 71 mg (4,61.10⁻⁴ mol) 1-hydroxybenzotriazole et 100 mg (5,07.10⁻⁴ mol) de 1-(2-méthoxyphényl)-pipérazine. Laisser revenir doucement à température ambiante. Après 24 h, évaporer le solvant. Reprendre par H₂O et extraire au CH₂Cl₂. Sécher sur MgSO₄.
Purifier sur colonne de silice. Eluant : Acétate d'éthyle : 100 %. On récupère 180 mg du composé du titre (solide blanc) que l'on recristallise dans l'éthanol.
Rendement : 95 %
Point de fusion : 100°C

### EXEMPLE 49 : 8-[(4-PHTALIMIDOBUTYL)OXY]THIOCHROMANE

Sous argon et sous agitation, dissoudre 500 mg (3,01 mmol) de 8-thiochromanol et 930 mg (3,31 mmol) de N-(4-bromobutyl)-phtalimide dans 4 cm de diméthylformamide (DMF). Ajouter 1,25 g (9,03 mmol/de K₂CO₃. Porter à 60°C pendant 6 h. Evaporer la DMF. Reprendre par H₂O. Extraire au CH₂Cl₂ et sécher sur MgSO₄. Après purification sur colonne de silce (éluant : CH₂Cl₂) on obtient 1,08 g le composé du titre (solide blanc).
Rendement : 98 %
Point de fusion : 116°C

### EXEMPLE 50 : 8-[(3-PHTALIMIDOPROPYL)OXY]THIOCHROMANE

en procédant selon l'exemple 49, mais en remplaçant la N-(4-bromobutyl)phtalimide par la N-(3-bromopropyl)phtalimide, on obtient le composé du titre.
Solvant de recristallisation : isopropanol
Rendement : 99 %
Point de fusion : 137°C

### EXEMPLE 51 : 8-[(2-PHTALIMIDOETHYL)OXY]THIOCHROMANE

En procédant selon l'exemple 49, mais en remplaçant le N-(4-bromobutyl)phtalimide par le N-(2-bromoéthyl)phtalimide, on obtient le composé du titre.
Point de fusion : 166°C

### EXEMPLE 52 : 8-{{3-[4-(2-METHOXYPHENYL)PIPERAZIN-1-YL]-2-METHYLPROPYL} OXY}THIOCHROMANE

### STADE A : 8-[(3-CHLORO-2-METHYLPROPYL)OXY]THIOCHROMANE

Sous argon, dissoudre 5 g (30,08 mmol) de thiochromanol dans 40 cm³ de diméthylformamide (DMF). Par petites portions ajouter 790 mg (33,08 mmol) d'hydrure de sodium. Après 30 min à 60°C, ajouter 12,89 g (75,19 mmol) de 1-bromo-3-chloro-2-méthylpropane dissous dans 10 cm³ de DMF. Porter à 60°C pendant 24 h.
Evaporer le solvant puis extraire au dichlorométhane après hydrolyse. Sécher sur MgSO₄. Après évaporation, passer le brut réactionnel sur colonne de silice normale. (Eluant : éther/éther de pétrole 5-95). On obtient 3,79 g d'une huile incolore qui contient le mélange du 8-[(3-chloro-2-méthylpropyl)oxy]thiochromane et de ses analogues bromé et éthylénique. Ce mélange est engagé sans autre purification dans le stade suivant.

### STADE B : 8-{{3-[4-(2-METHOXYPHENYL)PIPERAZIN-1-YL] 2-METHYLPROPYL} OXY}THIOCHROMANE

Sous argon, dissoudre 3,54 g de mélange obtenu au stade précédent dans 20 cm³ de DMF. Ajouter 2,67 g (20,68 mmol) de N,N-diisopropyléthylamine dissoute dans 5 cm³ de DMF et 3,98 g (20,08 mmol) de 1-(2-méthoxyphényl)pipérazine en solution dans 5 cm³ de DMF. Porter à 80°C pendant 24 h. Evaporer le solvant puis ajouter de l'eau. Extraire au dichlorométhane et sécher sur MgSO₄. Après évaporation, purifier sur colonne de silice normale. (Eluant : CH₂Cl₂ 100 % puis CH₂Cl₂/acétate d'éthyle 2 : 1), afin d'obtenir le composé du titre.
Rendement global : 20 %
Point de fusion (fumarate) : 189°C

### EXEMPLE 53 : 8-{[3-(4-PHENYLPIPERIDIN-1-YL)PROPYL]OXY}THIOCHROMANE

Sous argon, dissoudre 600 mg (2,09 mmol) de 8-[3-bromopropyl)oxy]thiochromane dans 6 cm³ d'acétonitrile. Ajouter la N,N-diisopropyléthylamine (405 mg ; 3,13 mmol) dissoute dans 5 cm³ d'acétonitrile puis la 4-phénylpipéridine (505 mg ; 3,13 mmol) dissoute dans 5 cm³ d'acétonitrile. Porter à reflux pendant une nuit. Evaporer l'acétonitrile. Reprendre le milieu réactionnel dans l'eau et extraire au dichlorométhane. Purifier sur colonne de silice flash. Eluant : méthanol/CH₂ Cl₂ 5 : 95.
On obtient le composé du titre (solide orangé).
Solvant de recristallisation : éthanol
Rendement global : 85 %
Point de fusion : 78-79°C

### EXEMPLES 54 A 56 :

En procédant comme dans l'exemple 53, on obtient les composés des exemples suivants à partir de l'amine appropriée :

### EXEMPLE 54 : 8-{[3-(4-PHENYLPIPERAZIN-1-YL)PROPYL]OXY}THIOCHROMANE

Solvant de cristallisation : éthanol
Rendement : 85 %
Point de fusion : 102°C

### EXEMPLE 55 : 8-{{3-[4-(4-FLUOROPHENYL)PIPERAZIN-1-YL]PROPYL}OXY} THIOCHROMANE

Rendement : 86 %
Point de fusion : 106°C

### EXEMPLE 56 : 8-{{3-[4-(DICYCLOPROPYLMETHYL)PIPERAZIN-1-YL]PROPYL}OXY} THIOCHROMANE

### EXEMPLE 57 : 8-{{3-[(5-METHOXY-DIHYDRO-[2H]-BENZO[b]PYRAN-3-YL)AMINO] PROPYL}OXY}THIOCHROMANE

Sous argon, dissoudre 1,6 g (5,58 mmol) de 8-[(3-bromopropyl)oxy]thiochromane, 1 g (5,58 mmol) de 3-amino-5-méthoxydihydro-[2H]-benzo[b]-pyrane, 1,69 g (16,74 mmol) de triéthylamine dans 20 cm³ de diméthylformamide. Maintenir à température ambiante pendant 24 h. Evaporer le solvant. Reprendre par H₂O et extraire au dichlorométhane. Sécher sur MgSO₄. Après évaporation, purifier sur colonne de silice flash. Eluant méthanol/CH₂Cl₂ : 5-95. On obtient le composé du titre (huile jaune)
Solvant de cristallisation : acétone/éthanol
Rendement global : 58 %
Point de fusion (fumarate) : 156-157°C

### EXEMPLE 58 : 8-{{4-[(5-METHOXY-DIHYDRO-[2H]-BENZO[b]PYRAN-3-YL)AMINO] BUTYL}OXY}THIOCHROMANE

En procédant comme dans l'exemple 57, mais en partant du 8-[(4-bromobutyl)oxy]thiochromane (stade A, exemple 2), on obtient le composé du titre.
Solvant de cristallisation : éthanol/acétone
Rendement : 54 %
Point de fusion : 176-177°C

### EXEMPLE 59 : 8-{[2-HYDROXY-3-(4-PHENYLPIPERIDIN-1-YL)PROPYL]OXY} THIOCHROMANE

En procédant selon l'exemple 1, mais en utilisant au stade B la 4-phénylpipéridine, on obtient le composé du titre.
Rendement : 97 %
Point de fusion : 104°C

### EXEMPLE 60 : 8-{[2-METHOXY-3-(4-PHENYLPIPERIDIN-1-YL)PROPYL]OXY} THIOCHROMANE

Sous argon, dissoudre 100 mg (2,60.10⁻⁴ mol) du composé de l'exemple 59 dans 2 cm³ de tétrahydrofurane (THF). Ajouter 10 mg (3,90.10⁻⁴ mol) d'hydrure de sodium. Laisser sous agitation 15 min à température ambiante puis additionner goutte à goutte 0,08 cm³ (185 mg ; 1,30 mmol) d'iodométhane. Laisser 1 h à température ambiante. Evaporer le THF puis reprendre doucement avec de l'eau. Extraire au dichlorométhane. Sécher sur MgSO₄. Purifier sur colonne de silice flash. Eluant : Acétate d'éthyle 100 %. On récupère le composé du titre (huile).
Rendement : 82 %
Point de fusion (fumarate) : 169°C

### EXEMPLE 61 A 64 :

En procédant comme dans les exemples 59 et 60, mais en utilisant l'amine appropriée, on obtient les composés des exemples suivants :

### EXEMPLE 61 : 8-{[2-HYDROXY-3-(4-PHENYLPIPERAZIN-1-YL)PROPYL]OXY} THIOCHROMANE

Rendement : 71 %
Point de fusion : 91°C

### EXEMPLE 62 : 8-{[2-METHOXY-3-(4-PHENYLPIPERAZIN-1-YL)PROPYL]OXY} THIOCHROMANE

Solvant de cristallisation : éthanol
Rendement : 79 %
Point de fusion : 86°C

### EXEMPLE 63 : 8-{{2-HYDROXY-3-[4-(DICYCLOPROPYLMETHYL)PIPERAZIN-1-YL] PROPYL}OXY}THIOCHROMANE

### EXEMPLE 64 : 8-{{2-METHOXY-3-[4-(DICYCLOPROPYLMETHYL)PIPERAZIN-1-YL] PROPYL}OXY}THIOCHROMANE

### EXEMPLE 65 A 68 :

En procédant selon l'exemple 1, mais en utilisant au stade B l'amine appropriée, on obtient les composés des exemples suivants :

### EXEMPLE 65 : 8-{{2-HYDROXY-3-[(4-PHENYLBUTYL)AMINO]PROPYL}OXY} THIOCHROMANE

Solvant de cristallisation : isopropanol
Rendement : 94 %
Point de fusion : 71°C

### EXEMPLE 66 : 8-{{2-HYDROXY-3-[(3-PHENYLPROPYL)AMINO]PROPYL}OXY} THIOCHROMANE

Solvant de cristallisation : éthanol
Rendement : 93 %
Point de fusion : 94°C

### EXEMPLE 67 : 8-{{3-[CYCLOOCTYL)AMINO]-2-HYDROXYPROPYL}OXY}THIOCHROMANE

Solvant de cristallisation : éthanol
Rendement : 94 %
Point de fusion : 97°C

### EXEMPLE 68 : 8-{{3-[(ADAMANTYL)AMINO]-2-HYDROXYPROPYL}OXY}THIOCHROMANE

Solvant de cristallisation : cyclohexane
Rendement : 81 %
Point de fusion : 92°C

### EXEMPLE 69 : 8-{{2-[N,N-bis-(4-PHTALIMIDOBUTYL)AMINO]ETHYL}OXY} THIOCHROMANE

### STADE A : 2-[(THIOCHROMAN-8-YL)OXY]ETHYLAMINE

Sous argon, dissoudre 2 g (12,03 mmol) de 8-thiochromanol dans 15 cm³ de DMF. Ajouter 5 g (36,09 mmol) de carbonate de potassium et une quantité catalytique d'iodure de potassium. Additionner alors goutte à goutte 5,45 g (72,18 mmol) de chloroacétonitrile mis en solution dans 5 cm³ de diméthylformamide (DMF). Chauffer à 60°C pendant 4 h puis évaporer le solvant. Reprendre par H₂O et extraire au dichlorométhane puis sécher sur MgSO₄. Après évaporation, purifier sur colonne de silice. Eluant Méthanol/CH₂Cl₂ 1 : 99. On obtient 2,44 g de 2-(thiochromane-8-yl-oxy)acétonitrile (Point de fusion : 66°C).

Dans un tricol sous argon, mettre en suspension 480 mg (12,67 mmol) d'hydrure double de lithium aluminium dans 20 cm³ d'éther anhydre. A l'aide d'une ampoule à brome, ajouter goutte à goutte 1 g (4,87 mmol) de nitrile obtenu précédemment dissous dans 20 cm³ d'éther anhydre. Après 5 mn à température ambiante, placer le tricol dans un bain de glace et hydrolyser lentement avec des morceaux de glace jusqu'à ce que les aluminates précipitent. Prélever la phase éthérée puis laver les sels plusieurs fois à l'éther. Réunir les phases éthérées. Les sécher sur MgSO₄ puis évaporer le solvant à froid. On obtient le composé du titre (huile incolore) qui n'a pas besoin d'être purifié.
Rendement : 93 %

### STADE B : 8-{{2-[N,N-bis-(4-PHTALIMIDOBUTYL)AMINO]ETHYL}OXY} THIOCHROMANE

Sous argon, dissoudre 200 mg (9.55.10⁻⁴ mol) de l'amine obtenue au stade A, 670 mg (2,39 mmol) de N-(4-bromobutyl)phtalimide, 185 mg (1,43 mmol) de N,N-diisopropyléthylamine dans 13 cm³ d'acétonitrile. Laisser 15 h à reflux puis évaporer le solvant. Reprendre par H₂O et extraire au dichlorométhane. Après évaporation, purifier sur colonne de silice. Eluant AcOEt/CH₂ Cl₂ 1-1. On obtient 340 mg du composé du titre (huile) qui précipite dans l'acétone.
Rendement : 58 %
Point de fusion : 66°C

### EXEMPLE 70 : 8-{{2-[N-(4-PHTALIMIDOBUTYL)AMINO]ETHYL}OXY}THIOCHROMANE

### STADE A : 2-[(THIOCHROMANE-8-YL)OXY]ETHYLAMINE

### STADE B : 8-{{2-[N-(4-PHTALIMIDOBUTYL)AMINO]ETHYL}OXY}THIOCHROMANE

Sous argon, dissoudre 1 g (4,78 mmol) de l'amine du stade A, 1,35 g (4,78 mmol) de N-(4-bromobutyl)phtalimide et 2 cm³ (1,45 g 14,33 mmol) de triéthylamine dans 20 cm³ de diméthylformamide (DMF).

Maintenir à température ambiante pendant 24 h. Evaporer le solvant. Reprendre par H₂O et extraire au dichlorométhane. Sécher sur MgSO₄. Après évaporation, purifier sur colonne de silice. Eluant : acétate d'éthyle méthanol : 9-1. On obtient le composé du titre (solide brun).
Solvant de recristallisation : éthanol
Rendement : 60 %
Point de fusion : 175°C

### EXEMPLE 71 : 8-{{3-[N-(5-METHOXY-DIHYDRO-[2H]-BENZO[b]PYRAN-3-YL)-N-PROPYLAMINO]PROPYL}OXY}THIOCHROMANE

Point de fusion (fumarate) : 79°C
Solvant de cristallisation : cyclohexane

### EXAMPLE 72 : 8-{{2-METHOXY-3-[(4-PHENYLBUTYL)AMINO]PROPYL}OXY} THIOCHROMANE

Point de fusion (fumarate) : 106°C
Solvant de cristallisation : acétone/éthanol

### ETUDE PHARMACOLOGIQUE DES COMPOSES DE L'INVENTION

### EXEMPLE A : ETUDE DE LA TOXICITE AIGUE

La toxicité aiguë a été effectuée après administration orale à des lots de cinq souris (20 ± 2 grammes) de doses croissantes (0,1 - 0,25 - 0,50 - 0,75 - 1g/kg-¹) des produits de l'invention.
Les animaux ont été observés à intervalles réguliers au cours de la première journée et quotidiennement pendant les deux semaines suivant le traitement. Il apparaît que les composés de l'invention sont totalement atoxiques. Aucun décès n'est observé après administration d'une dose de 1 g.kg-¹. On ne constate pas de troubles après administration de cette dose.

### EXEMPLE B : MESURE DE L'AFFINITE DES COMPOSES DE L'INVENTION POUR LES RECEPTEURS 5HT_{1A}.

### PROTOCOLE :

L'affinité in-vitro des composés de l'invention, pour les récepteurs sérotoninergiques 5-HT_{1A}, a été déterminée par la mesure du déplacement de la (3H) 8-hydroxy-2-(N,N-dipropylamino)tétraline [ou (3H) 8-OH-DPAT], agoniste sélectif de ce récepteur, sur des préparations d'hippocampe de rat.

### RESULTATS :

Les composés de formule générale (I) se révèlent être de très puissants ligands des récepteurs 5-HT_{1A}, avec des constantes d'affinité de l'ordre du nanomolaire.

### EXEMPLE C : MESURE DE L'AFFINITE DES COMPOSES DE L'INVENTION POUR LES RECEPTEURS β₁, β₂, D₁, D₂, 5-HT_{1C}, 5-HT_{1D}, 5-HT₂ ET 5-HT₃.

### PROTOCOLE :

L'affinité in-vitro des composés de l'invention a été déterminée :
- pour les récepteurs adrénergiques β₁, par la mesure du déplacement du dihydroalprénolol, sur des préparations de cortex frontal de rat,
- pour les récepteurs adrénergiques β₂, par la mesure du déplacement du dihydroalprénolol, sur des préparations de parenchyme pulmonaire de rat,
- pour les récepteurs dopaminergiques D₁, par la mesure du déplacement du SCH 23390, sur des préparations de striatum de rat,
- pour les récepteurs dopaminergiques D₂, par la mesure du déplacement du Raclopride, sur des préparations de striatum de rat,
- pour les récepteurs sérotoninergiques 5-HT_{1C}, par la mesure du déplacement du N-méthylmésulergine, sur des préparations de cortex frontal et d'hippocampe de rat,
- pour les récepteurs sérotoninergiques 5-HT_{1D}, par la mesure du déplacement de la 5-OH-tryptamine, sur des préparations de cortex, striatum et globus pallidus de rat,
- pour les récepteurs sérotoninergiques 5-HT₂, par la mesure du déplacement de l'amino-iodo-kétansérine, sur des préparations de cortex frontal de rat,
- pour les récepteurs sérotoninergiques 5-HT₃, par la mesure du déplacement du BRL 43694, sur des préparations d'area postrema de rat.

### RESULTATS :

Certains composés de l'invention présentent une affinité pour les récepteurs β₁, β₂, D₁, D₂, 5-HT_{1C}, 5-HT_{1D}, 5-HT₂ et 5-HT₃ nettement plus faible que pour les récepteurs 5-HT_{1A}. Les composés de l'invention sont donc des ligands sélectifs des récepteurs 5-HT_{1A}.

### EXEMPLE D : EVALUATION DE L' ACTIVITE ANTAGONISTE DES RECEPTEURS 5HT_{1A} DES COMPOSES DE L'INVENTION

### PROTOCOLE :

L'évaluation de l'activité antagoniste des récepteurs 5HT_{1A}, des composés de l'invention, a été faite en stimulant, en présence du composé à tester, l'adénylate cyclase par 10 »M de forskoline, en absence ou en présence de 0,1 »M de [8-hydroxy-2-(di-n-propylamino)tétraline] (8-OH-DPAT).

Les produits de l'invention ont été testés pour des gammes de concentrations allant de 10 nM à 10 »M.

### RESULTATS :

Les composés de l'invention s'opposent de façon compétitive (IC₅₀ < 50 nM) à l'inhibition de l'adénylate cyclase, provoquée par la 8-OH-DPAT (0,1 »M), dans des homogénats d'hippocampe de rat, ce qui traduit une forte activité antagoniste des récepteurs 5HT_{1A}.

### EXEMPLE E : ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'évènements aversifs incontrôlables, un déficit lors des tâches d'évitement ultérieures.

### PROTOCOLE :

Nous utilisons des rats mâles Wistar d'un poids compris entre 180 et 200 grammes. Les animaux sont maintenus à l'animalerie une semaine avant le test, dans des boîtes en plastique, par groupes de 10, à une température ambiante de 21° C ∓ 1° C, avec libre accès à l'eau et à la nourriture.
Les animaux sont ensuite isolés dans des boîtes de petites dimensions et sont soumis à 60 chocs électriques inévitables (0,8 mA toutes les minutes ∓ 15 secondes). Un groupe de rats témoins ne reçoit pas de chocs électriques.
La capacité des animaux à réaliser un apprentissage d'évitement (passage d'un compartiment à l'autre, afin d'éviter les chocs électriques) est appréciée 48 heures après et pendant 3 jours consécutifs. Lors des séances d'apprentissage, les animaux subissent 2 essais par minute pendant 15 minutes. Le nombre d'échecs d'échappement est noté pour chaque rat. Les animaux sont traités (i.p. ; 0.5 cm³/100 g) 6 heures après les chocs inévitables et 4 jours de suite, le matin 30 minutes avant la séance d'apprentissage et le soir entre 18 et 19 h.
Les produits étudiés sont mis en solution dans de l'eau distillée.
Les produits étudiés sont administrés aux doses 0.25 mg.kg/jour.

### RESULTATS :

Le test prouve que les produits de l'invention diminuent significativement le nombre d'échecs d'échappement ce qui traduit, pour les produits de l'invention, une forte activité de type antidépressive.

### EXEMPLE F : ETUDE DE L'ACTIVITE ANXIOLYTIQUE - TEST DIT DES CAGES CLAIRE/OBSCURE CHEZ LA SOURIS

### PRINCIPE :

Nous nous proposons d'étudier les effets anxiolytiques des composés de l'invention, au moyen du test dit des cages claires/obscures chez la souris.

### PROTOCOLE :

Ce test a été mis au point par Crawley et al. (1981, Pharmacol. Biochem. Behav.), puis modifié et comportementalement validé.

Il s'agit de deux cages de taille égale (20×20×14 cm) en PVC. L'une est fortement éclairée par une lampe de 100 W(lumière "froide"), l'autre est obscurcie. Les deux cages sont séparées l'une de l'autre au moyen d'un petit tunnel opaque (5×7 cm). Les souris sont individuellement introduites dans la cage éclairée et dès qu'elles ont pénétré pour la première fois dans la cage obscure, on enregistre, au moyen de claviers reliés à un ordinateur, durant 5 minutes, le temps passé par les animaux dans la cage éclairée ainsi que le nombre des transitions entre la cage obscure et la cage éclairée.

Chaque groupe expérimental comprend au minimum 15 animaux.

### RESULTATS :

L'administration, par voie intrapéritonéale de certains produits de l'invention entraine une augmentation simultanée du temps passé par les souris dans la cage éclairée et du nombre des transitions entre la cage obscure et la cage éclairée.

Cette augmentation significative des deux paramètres étudiés montrent la remarquable activité anxiolytique de certains composés de l'invention.

### EXEMPLE G : COMPOSITION PHARMACEUTIQUE

Comprimés dosés à 5 mg de 8-{{3-[4-(2-méthoxyphényl)pipérazin-1-yl] 2-hydroxypropyl}oxy}thiochromane

Formule pour 10 000 comprimés :

| | |
|---|---|
| 8-{{3-[4-(2-méthoxyphényl)pipérazin-1-yl] 2-hydroxypropyl}oxy}thiochromane | 50 g |
| Amidon de blé | 75 g |
| Amidon de maïs | 75 g |
| Lactose | 325 g |
| Stéarate de magnésium | 10 g |
| Silice | 5 g |
| Hydroxypropyl cellulose | 10 g |

## Revendications

1. Composés de formule (I) : 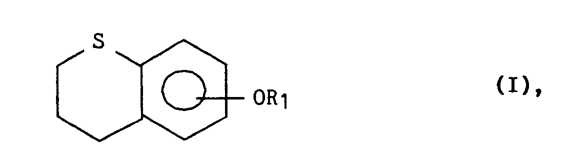 dans laquelle :
- R₁ représente :
* un groupement de formule (A) : 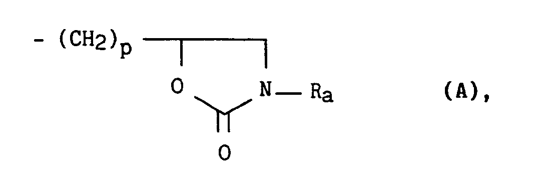 dans laquelle Rₐ représente un atome d'hydrogène ou un radical alkyle inférieur et p est égal à 1, 2 ou 3,
* ou un groupement de formule (B) : 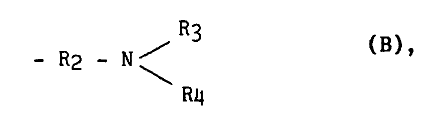 dans laquelle
→ R₂ est un groupement choisi parmi :
- -R'₂ - où R'₂ représente un groupement -(CH₂)ₙ- ou 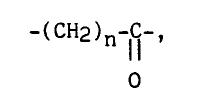 avec n représentant un nombre entier de 1 à 6, R'₂ étant non substitué ou substitué sur la partie alkylène par un radical alkyle inférieur, aryle, ou arylalkyle inférieur,
- et 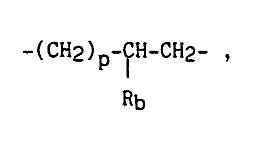 où p est égal à 1, 2 ou 3 et où R_{b} représente un radical hydroxyle, alkoxy inférieur, alkylcarbonyloxy inférieur, aryloxy, ou (arylalkyle inférieur)oxy,
→ R₃ et R₄, identiques ou différents, représentent, chacun indépendamment l'un de l'autre,
- un atome d'hydrogène,
- un radical alkyle inférieur, non substitué ou substitué par un ou plusieurs radicaux choisis parmi halogène, hydroxyle et alcoxy inférieur,
- un radical cycloalkyle, cycloalkylalkyle inférieur, di(cycloalkyl)-alkyle inférieur, non substitué ou substitué sur les parties "cycloalkyle" par un ou plusieurs radicaux choisis parmi halogène, oxo, alkyle inférieur et alkoxy inférieur,
- un radical acyle, non substitué ou substitué par un ou plusieurs radicaux choisis parmi halogène, alkyle inférieur et alkoxy inférieur,
- un radical choisi parmi adamantyle, benzopyranyle, benzofuryle et aryle ; ce radical étant non substitué ou substitué par un ou plusieurs radicaux choisis parmi halogène, hydroxyle, alkyle inférieur, alkoxy inférieur et trifluorométhyle,
- un radical arylalkyle inférieur, non substitué ou substitué sur le noyau aryle par un ou plusieurs radicaux choisis parmi halogène, hydroxyle, alkyle inférieur, alkoxy inférieur, et trifluorométhyle,
- un radical 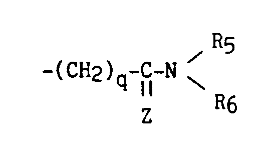 dans lequel q représente 0 ou un nombre entier de 1 à 4 et Z représente un atome d'oxygène ou 2 atomes d'hydrogène,
et dans lesquels R₅ et R₆ représentent, chacun indépendamment l'un de l'autre, un atome d'hydrogène, un alkyle inférieur, un acyle, un aryle non substitué ou substitué par un ou plusieurs radicaux choisis parmi halogène, hydroxyle, alkyle inférieur, alkoxy inférieur, et trifluorométhyle ; ou un arylalkyle inférieur non substitué ou substitué sur le noyau aryle par un ou plusieurs radicaux choisis parmi halogène, hydroxyle, alkyle inférieur, alkoxy inférieur, et trifluorométhyle,
ou R₅ et R₆ forment ensemble avec l'atome d'azote qui les porte soit un hétérocycle, non substitué ou substitué, choisi parmi pyrrolidine, pipéridine, pipérazine, morpholine, thiomorpholine, homopipérazine, homopipéridine, phtalimide, et azabicycloalkyle contenant de 7 à 12 sommets, soit un groupement (azaspiroalkyl)alkyle inférieur, le système azaspiroalkyle comprenant de 6 à 12 sommets carbonés, et étant non substitué ou substitué par un ou plusieurs radicaux choisis parmi halogène, oxo, et alkyle inférieur,
avec la réserve que lorsque R₂ représente un
groupement 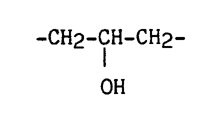 et simultanément R₃ est un atome d'hydrogène, alors R₄ ne peut pas représenter un atome d'hydrogène, un radical alkyle inférieur, ou un radical cycloalkyle,
→ ou R₃ et R₄ forment avec l'atome d'azote qui les porte :
- soit un hétérocycle, non substitué ou substitué, choisi parmi pyrrolidine, pipéridine, pipérazine, morpholine, thiomorpholine, homopipérazine, homopipéridine, phtalimide, et azabicycloalkyle contenant de 7 à 12 sommets,
avec la réserve que si R₂ représente un groupement -(CH₂)ₙ- avec n tel que défini précédemment alors R₃ et R₄ ne peuvent pas former avec l'atome d'azote qui les porte un groupement pipéridino non substitué ou substitué par un ou plusieurs radicaux alkyles inférieurs ou alkoxy inférieurs,
- soit un groupement azaspiroalkyle de 6 à 12 sommets, non substitué ou substitué par un ou plusieurs radicaux choisis parmi halogène, oxo, alkyle inférieur, et alkoxy inférieur, étant entendu que lorsque R₂ représente un groupement 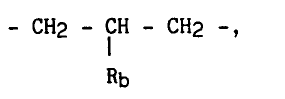 R₃ et R₄ ne peuvent pas former avec l'atome d'azote qui les porte une pipérazine substituée en position 4 par un radical diphénylméthyle non substitué ou substitué sur les noyaux phényles par un ou plusieurs radicaux choisis parmi halogène, alkyle inférieur, alkoxy inférieur et trifluorométhyle,
étant entendu que,:
. l'expression "substitué" affectée au terme "hétérocycle" signifie que l'hétérocycle est substitué par un ou plusieurs radicaux choisis parmi :
- halogène,
- hydroxyle,
- oxo,
- alkyle inférieur,
- alkoxy inférieur,
- alkoxycarbonyle inférieur,
- et les groupements -(CH₂)_{n'}-E, 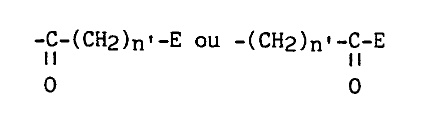 avec n' représentant 0 ou un nombre entier de 1 à 4 et E représentant un radical choisi parmi aryle, benzhydryle, thiényle, pyrrolyle, pyrrolidinyle, furyle, pyrimidinyle, pyridyle, cycloalkyle et dicycloalkylalkyle inférieur, le groupement E pouvant être non substitué ou substitué par un ou plusieurs groupements choisis parmi halogène, hydroxyle, alkyle inférieur, alkoxy inférieur, et trifluorométhyle,
. le terme "cycloalkyle" représente un groupement cyclique de 3 à 8 atomes de carbone,
. le terme "acyle" représente un groupement alkylcarbonyle inférieur, arylcarbonyle, et arylalkylcarbonyle inférieur,
. le terme "aryle" représente un groupement choisi parmi phényle et naphtyle,
. et les termes "alkyle inférieur" et "alkoxy inférieur" signifient des groupements, linéaires ou ramifiés, contenant de 1 à 6 atomes de carbone,
leurs isomères optiques, sous forme pure ou sous forme de mélange, ainsi que leurs sels d'addition à un acide ou une base pharmaceutiquement acceptable.

2. Composés selon la revendication 1 pour lesquels R₃ et R₄ forment avec l'atome d'azote qui les porte une pipérazine non substituée ou substituée en position 4 par un radical choisi parmi :
- alkyle inférieur,
- alkoxy inférieur,
- alkoxycarbonyle inférieur
- et les groupements -(CH₂)_{n'}-E, 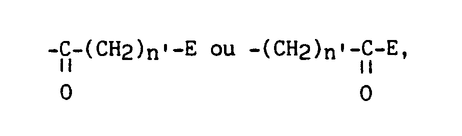 avec n' représentant 0 ou un nombre entier de 1 à 4 et E représentant un radical choisi parmi aryle, benzhydrile, thiényle, pyrrolyle, pyrrolidinyle, furyle, pyrimidinyle, pyridyle, cycloalkyle et dicycloalkylalkyle inférieur, le groupement E pouvant être non substitué ou substitué par un ou plusieurs groupements choisis parmi halogène, oxo, hydroxyle, alkyle inférieur, alkoxy inférieur, et trifluorométhyle,
leurs isomères optiques, et leur sels d'addition à un acide ou à une base pharmaceutiquement acceptable.

3. Composés selon la revendication 1 pour lesquels R₂ représente un groupement -(CH₂)ₙ- ou 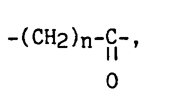 avec n représentant un nombre entier de 1 à 6, les groupements -(CH₂)ₙ- et 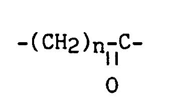 pouvant être substitués sur leur partie alkylène par un radical alkyle inférieur, aryle, ou arylalkyle inférieur,
leurs isomères optiques, et leur sels d'addition à un acide ou à une base pharmaceutiquement acceptable.

4. Composés selon la revendication 1 pour lesquels R₂ représente un groupement 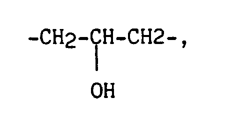 leurs isomères optiques, ainsi que leur sels d'addition à un acide ou à une base pharmaceutiquement acceptable.

5. Composé selon la revendication 1 qui est le 8-{{3-[4-(2-méthoxyphényl)pipérazin-1-yl]-2-hydroxypropyl}oxy}thiochromane, ses isomères optiques ainsi que ses sels d'addition à un acide pharmaceutiquement acceptable.

6. Composé selon la revendication 1 qui est le 8-{{4-[4-(2-méthoxyphényl)pipérazin-1-yl]butyl}oxy}thiochromane ainsi que ses sels d'addition à un acide pharmaceutiquement acceptable.

7. Composé selon la revendication 1 qui est le 8-{{4-[4-(3-trifluorométhylphényl)pipérazin-1-yl]butyl}oxy}thiochromane ainsi que ses sels d'addition à un acide pharmaceutiquement acceptable.

8. Composé selon la revendication 1 qui est le 8-{{3-{N,N-di[4-(7,9-dioxo-8-azaspiro[4,5]décan-8-yl)but-1-yl]amino}-2-hydroxypropyl}oxy} thiochromane, ses isomères optiques ainsi que ses sels d'addition à un acide pharmaceutiquement acceptable.

9. Composé selon la revendication 1 qui est le 8-{[(2-oxo 3-tertiobutyl oxazolidin-5-yl)méthyl]oxy}thiochromane ainsi que ses isomères optiques et ses sels d'addition à un acide pharmaceutiquement acceptable.

10. Composé selon la revendication 1 qui est le 8-{[4-(7,9-dioxo-8-azaspiro[4,5]décan-8-yl)butyl]oxy}thiochromane ainsi que ses sels d'addition à un acide pharmaceutiquement acceptable.

11. Procédé de préparation des composés de formule (I) selon la revendication 1, caractérisé en ce que l'on utilise comme matière première un thiochromanol de formule (II) : 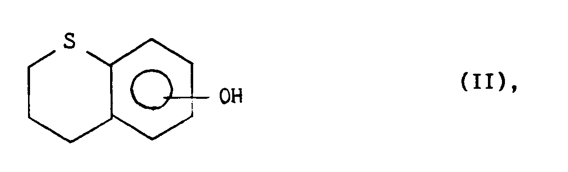 que l'on fait réagir :
- soit avec un composé di-halogéné de formule (IIₐ) :
X₁-R'₂-Y'₁ **(II**_{**a**}**),**
dans laquelle R'₂ est tel que défini dans la revendication 1, X₁ représente un atome d'halogène et Y'₁ représente un atome d'halogène, ou un radical (C₁-C₄) alkoxy ou un hydroxyle lorsque R'₂ contient une fonction carbonyle, pour donner un composé de formule (II/b) : 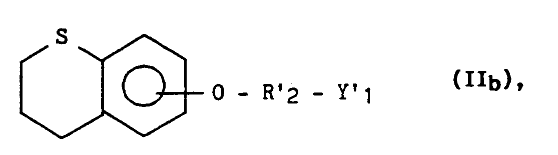 dans laquelle R'₂ et Y'₁ sont tels que définis précédemment, que l'on fait réagir, après une hydrolyse de la fonction ester lorsque Y₁ représente un radical (C₁-C₄)alkoxy, avec une amine de formule (II_{c}) : 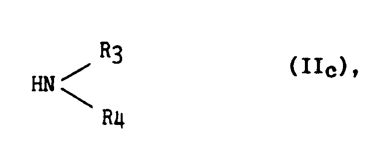 dans laquelle R₃ et R₄ sont tels que définis dans la revendication 1, pour donner un composé de formule (I₁) : 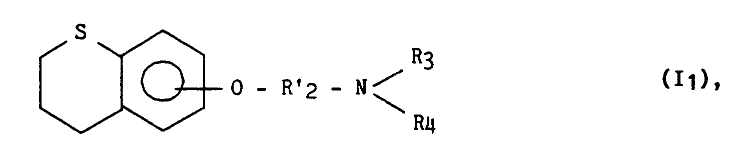 dans laquelle R'₂, R₃, et R₄ sont tels que définis précédemment,
cas particulier des composés de formule (I) où R₁ représente un groupement 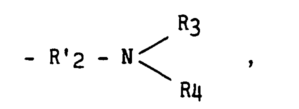 où R'₂, R₃ et R₄ sont tels que définis précédemment,
- soit avec un composé de formule (II_{d}) ou (II_{d'}) : 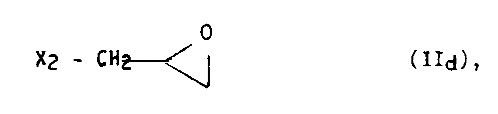
X₂―(CH₂)ₚ―CH=CH₂ **(II**_{**d'**}**),**
où X₂ représente un atome d'halogène, et où p est tel que défini dans la formule (I) pour donner, aprés traitement par un peracide lorsqu'on a utilisé un réactif de formule (II/d'), un composé de formule (IIₑ) : 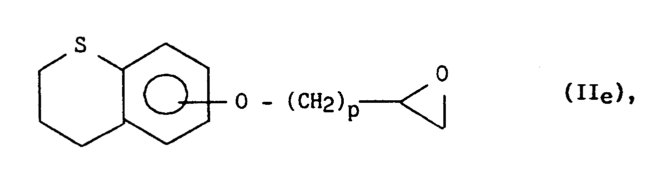 où p est tel que défini précédemment,
composé de formule (IIe) que l'on fait réagir :
* ou bien avec une amine de formule (II_{c}) : 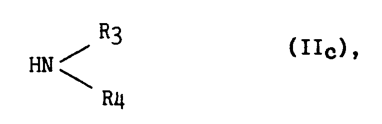 dans laquelle R₃ et R₄ sont tels que définis précédemment,
pour donner un composé de formule (I₂) : 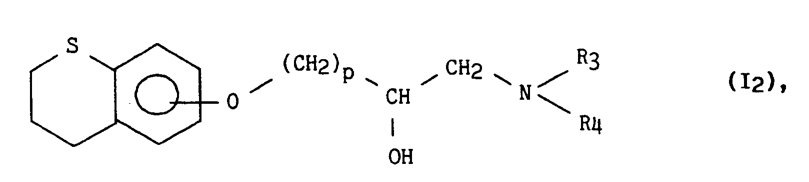 dans laquelle, p, R₃ et R₄ sont tels que définis précédemment, cas particulier des composés de formule (I) selon la revendication 1, dans lequel R₁ représente un groupement : 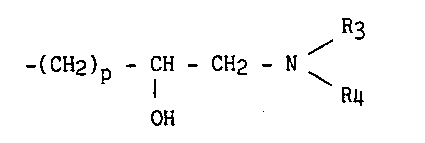 où p, R₃ et R₄ sont tels que définis précédemment,
composes de formule (I₂) que l'on substitue sur le groupement hydroxyle situé en β de l'atome d'azote porteur des radicaux R₃ et R₄, par réaction avec un composé de formule (II_{f}) :
X3 - R_{β} **(II**_{**f**}**)**
dans lesquelles X₃ représente un atome d'halogène et R_{β} est un radical alkyle inférieur, alkylcarbonyle inférieur, aryle, ou arylalkyle inférieur avec les termes "alkyle inférieur", et "aryle" tels que définis dans la revendication 1, réaction éventuellement accompagnée d'une étape de protection de l'azote portant R₃ et R₄, pour donner un produit de formule (I₃) : 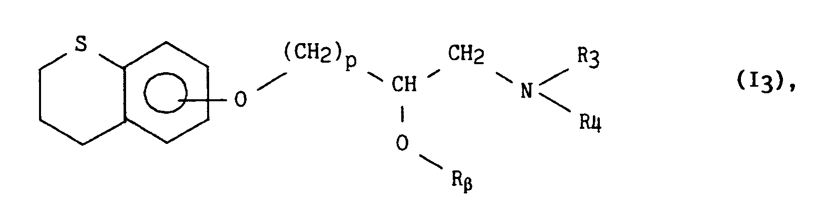 où p, R₃, R₄ et R_{β} sont tels que définis précédemment,
cas particulier des composés de formule générale (I) selon la revendication 1, où R₁ représente un groupement : 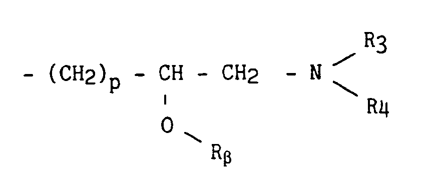 où p, R₃, R₄ et R_{β} sont tels que définis précédemment,
* ou bien avec de l'azidure de sodium, pour donner un composé de formule (IIg) : 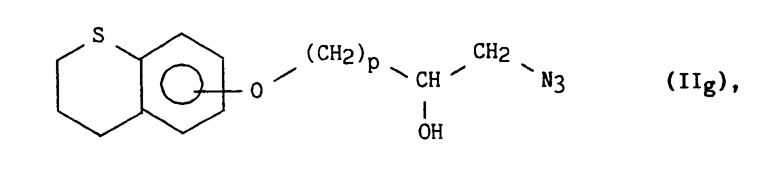 où p est tel que défini précédemment, que l'on fait ensuite réagir avec de l'éthanol et un catalyseur de Lindlar, afin d'obtenir une amine primaire de formule (I₄) : 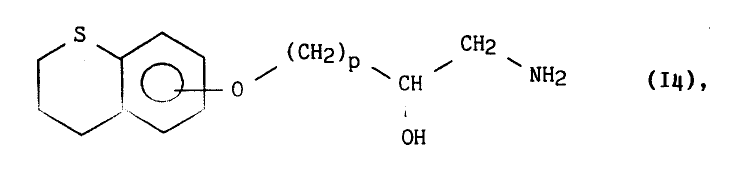 où p est tel que défini précédemment, cas particulier des composés de formule générale (I₂), où R₁ représente un groupement : 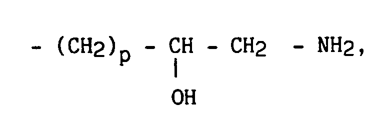 où p est tel que défini précédemment,
composé de formule (I₄) que l'on fait réagir avec du N,N'-carbonyldiimidazole, pour obtenir un composé de formule (I₅) : 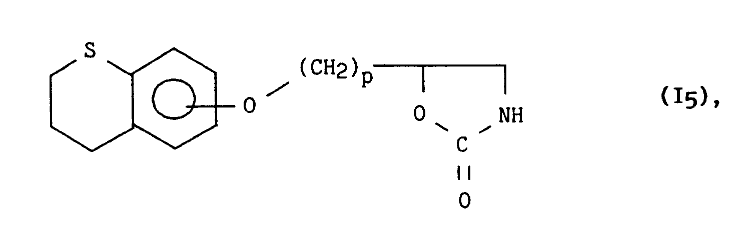 dans laquelle p est tel que défini précédemment, cas particulier des composés de formule (I) selon la revendication 1 où R₁ représente un groupement de formule : 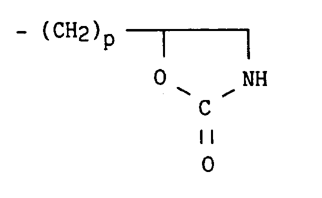 dans lequel p est tel que défini précédemment,
que l'on alkyle, le cas échéant, par l'intermédiaire d'un halogénoalkyle inférieur de formule (IIₕ) :
Rₐ' - X₄ **(II**_{**h**}**),**
où Rₐ' est un radical alkyle inférieur et où X₄ est un atome d'halogène,
pour donner un composé de formule (I₆) : 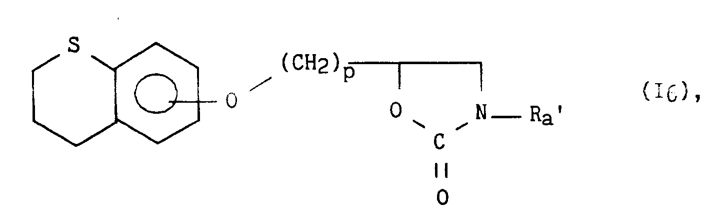 dans laquelle p et Rₐ' sont tels que définis précédemment,
cas particulier des composés de formule (I) selon la revendication 1 où R₁ représente un groupement de formule : 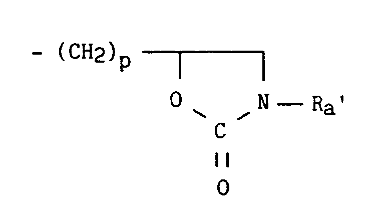 dans laquelle p et Rₐ' sont tels que définis précédemment,
les composés de formule (I₁), (I₂), (I₃), (I₄), (I₅) et (I₆) constituent l'ensemble des composés de formule (I) selon la revendication 1 qui peuvent être, si on le désire,
- alkylés, si on est en présence d'une amine secondaire, sur l'azote portant les substituants R₃ et R₄,
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- ou salifiés, par un acide ou une base pharmaceutiquement acceptable.

12. Procédé de préparation des composés de formule (I₇) et (I_{7'}), cas particulier des composés de formule (I) selon la revendication 1 : 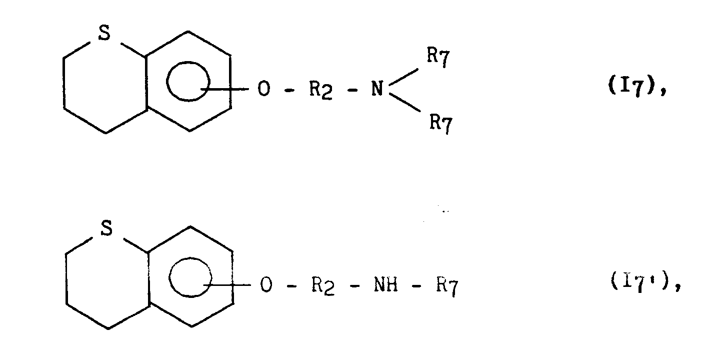 dans lesquelles R₂ est tel que défini dans la revendication 1 et R₇ représente :
- un radical alkyle inférieur, non substitué ou substitué par un ou plusieurs radicaux choisis parmi halogène, hydroxyle et alcoxy inférieur,
- un radical cycloalkyle, cycloalkylalkyle inférieur, di(cycloalkyl)alkyle inférieur, non substitué ou substitué, sur les parties "cycloalkyle", par un ou plusieurs radicaux choisis parmi halogène, oxo, alkyle inférieur et alkoxy inférieur,
- un radical acyle, non substitué ou substitué par un ou plusieurs radicaux choisis parmi halogène, alkyle inférieur et alkoxy inférieur,
- un radical choisi parmi adamantyle, benzopyranyle, benzofuryle, et aryle, non substitué ou substitué par un ou plusieurs radicaux choisis parmi halogène, alkyle inférieur, alkoxy inférieur et trifluorométhyle,
- un radical arylalkyle inférieur, non substitué ou substitué sur le noyau aryle par un ou plusieurs radicaux choisis parmi halogène, alkyle inférieur, alkoxy inférieur et trifluorométhyle,
- ou un radical 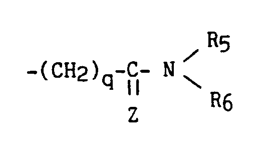 dans lequel q représente 0 ou un nombre entier de 1 à 4, et Z représente un atome d'oxygène ou 2 atome d'hydrogène,
et dans lesquels R₅ et R₆ sont tels que définis dans la revendication 1,
les termes "alkyle inférieur", "alcoxy inférieur", "cycloalkyle", "acyle", et "aryle", étant tels que définis dans la revendication 1,
caractérisé en ce que l'on fait réagir un composé de formule (I₈), 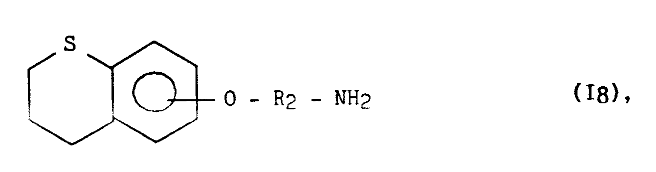 où R₂ est tel que défini précédemment,
avec un composé halogéné de formule (IIᵢ) :
X₅ - R₇ **(II**_{**i**}**),**
dans laquelle R₇ est tel que défini précédemment et X₅ représente un atome d'halogène,
pour donner suivant la stoechiométrie utilisée un composé de formule (I₇) ou (I_{7'}) tels que définis précédemment,
composés de formule (I₇) et (I_{7'}) qui peuvent être, si on le désire,
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- ou salifiés, par un acide ou une base pharmaceutiquement acceptable.

13. Procédé de préparation des composés de formule (I₁) : 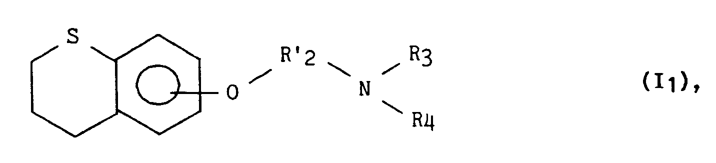 dans lequel R'₂, R₃ et R₄ sont tels que définis dans la revendication 1,
cas particulier des composés de formule (I) selon la revendication 1, où R₁ représente un groupement de formule : 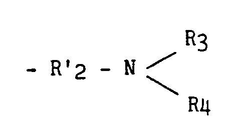 dans laquelle R'₂, R₃ et R₄ sont tels que définis précédemment,
caractérisé en ce que :
on fait réagir un thiochromanol de formule (II) : 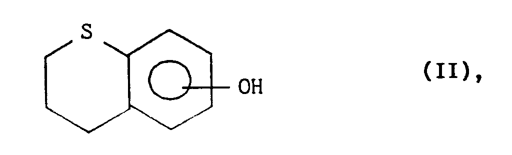 avec une halogénoalkylamine de formule (IIⱼ) : 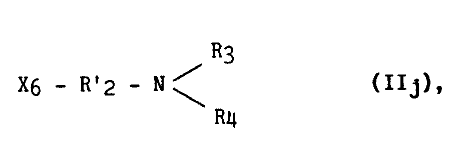 où R'₂, R₃ et R₄ sont tels que définis précédemment et où X₆ représente un atome d'halogène, pour donner un composé de formule (I₁) tel que défini précédemment,
composés de formule (I₁) qui peuvent être, si on le désire,
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- ou salifiés, par un acide ou une base pharmaceutiquement acceptable.

14. Procédé de préparation des composés de formule (I₆), cas particuliers des composés de formule (I) selon la revendication 1 : 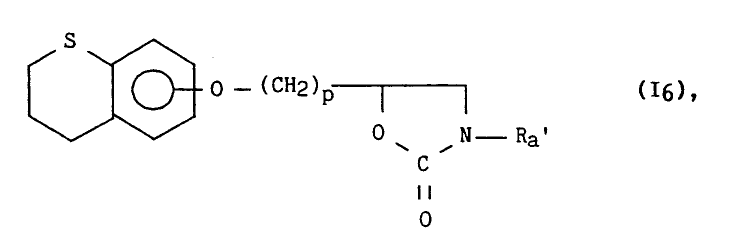 où p est tel que défini dans la revendication 1 et Rₐ' représente un radical alkyle inférieur,
caractérisé en ce que l'on fait réagir un composé de formule (IIₖ) : 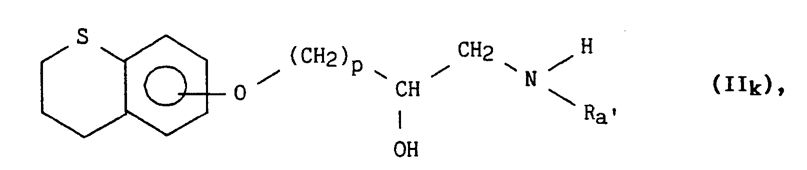 dans laquelle p et Ra' sont tels que définis précédemment, cas particulier des composés de formule (I) selon la revendication 1 dans lesquels R₁ représente un groupement 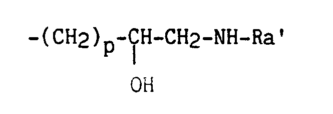 dans lequel Ra' et p sont tels que définis précédemment, avec du N,N'-carbonyldiimidazole pour obtenir un composé de formule (I₆), tel que défini précédemment,
cas particulier des composés de formule (I) selon la revendication 1, où R₁ représente un groupement de formule : 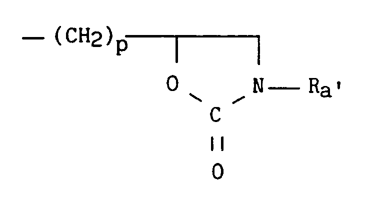 dans laquelle R_{a'} et p sont tels que définis précédemment,
composés de formule (I₆) qui peuvent être, si on le désire,
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs isomères optiques.

15. Composition pharmaceutique contenant comme principe actif au moins un composé selon la revendication 1 ou un de ses sels d'addition à un acide ou à une base pharmaceutiquement acceptable, en combinaison avec un ou plusieurs excipients ou véhicules pharmaceutiquement acceptables.

16. Composition pharmaceutique contenant comme principe actif au moins un composé selon la revendication 1 ou un de ses sels d'addition à un acide ou à une base pharmaceutiquement acceptable, associé à au moins un principe actif antidépresseur connu, en combinaison avec un ou plusieurs excipients ou véhicules pharmaceutiquement acceptables.

17. Composition pharmaceutique selon les revendications 15 ou 16 utile dans le traitement et la prévention du stress, de l'anxiété, de la dépression, des psychoses, de la schizophrénie, de la douleur et des troubles du comportement alimentaire et sexuel.

18. Composition pharmaceutique selon les revendications 15 ou 16 utile dans le traitement de la dépression.

## Claims

1. Compounds of formula (I): 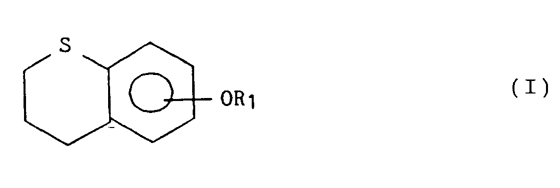 in which:
- R₁ represents:
* a group of formula (A): 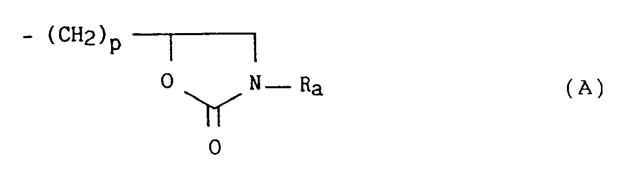 in which Rₐ represents a hydrogen atom or a lower alkyl radical and p is 1, 2 or 3,
* or a group of formula (B): 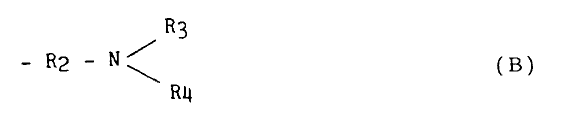 in which
→ R₂ is a group selected from:
- -R'₂-, wherein R'₂ represents a group -(CH₂)ₙ- or 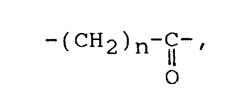 wherein n represents an integer from 1 to 6, R'₂ being unsubstituted or substituted in the alkylene moiety by a lower alkyl, aryl or aryl-lower alkyl radical,
- and 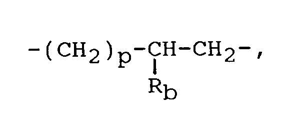 wherein p is 1, 2 or 3 and R_{b} represents a hydroxy, lower alkoxy, lower alkylcarbonyloxy, aryloxy or aryl-lower alkoxy radical,
→ R₃ and R₄, which are identical or different, each independently of the other represents
- a hydrogen atom,
- a lower alkyl radical that is unsubstituted or substituted by one or more radicals selected from halogen, hydroxy and lower alkoxy,
- a cycloalkyl, cycloalkyl-lower alkyl or di(cycloalkyl)-lower alkyl radical each of which is unsubstituted or substituted in the cycloalkyl moiety/moieties by one or more radicals selected from halogen, oxo, lower alkyl and lower alkoxy,
- an acyl radical that is unsubstituted or substituted by one or more radicals selected from halogen, lower alkyl and lower alkoxy,
- a radical selected from adamantyl, benzopyranyl, benzofuryl and aryl, that radical being unsubstituted or substituted by one or more radicals selected from halogen, hydroxy, lower alkyl, lower alkoxy and trifluoromethyl,
- an aryl-lower alkyl radical that is unsubstituted or substituted in the aryl nucleus by one or more radicals selected from halogen, hydroxy, lower alkyl, lower alkoxy and trifluoromethyl,
- a radical 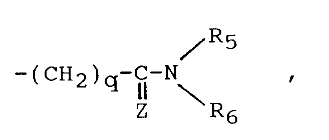 in which q represents 0 or an integer from 1 to 4 and Z represents an oxygen atom or two hydrogen atoms,
and in which R₅ and R₆ each independently of the other represents a hydrogen atom, a lower alkyl radical, an acyl radical, an aryl radical that is unsubstituted or substituted by one or more radicals selected from halogen, hydroxy, lower alkyl, lower alkoxy and trifluoromethyl; or an aryl-lower alkyl radical that is unsubstituted or substituted in the aryl nucleus by one or more radicals selected from halogen, hydroxy, lower alkyl, lower alkoxy and trifluoromethyl,
or R₅ and R₆, together with the nitrogen atom carrying them, form either an unsubstituted or substituted heterocycle selected from pyrrolidine, piperidine, piperazine, morpholine, thiomorpholine, homopiperazine, homopiperidine, phthalimide, and azabicycloalkyl containing from 7 to 12 ring members, or an azaspiroalkyl-lower alkyl group, the azaspiroalkyl system containing from 6 to 12 carbon ring members and being unsubstituted or substituted by one or more radicals selected from halogen, oxo and lower alkyl,
with the proviso that when R₂ represents a group 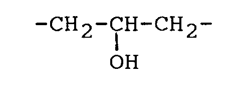 and R₃ at the same time is a hydrogen atom, R₄ may not represent a hydrogen atom, a lower alkyl radical or a cycloalkyl radical,
→ or R₃ and R₄, together with the nitrogen atom carrying them, form:
- either an unsubstituted or substituted heterocycle selected from pyrrolidine, piperidine, piperazine, morpholine, thiomorpholine, homopiperazine, homopiperidine, phthalimide, and azabicycloalkyl containing from 7 to 12 ring members,
with the proviso that when R₂ represents a group -(CH₂)ₙ-, wherein n is as defined above, R₃ and R₄ may not form, together with the nitrogen atom carrying them, a piperidino group that is unsubstituted or substituted by one or more lower alkyl or lower alkoxy radicals,
- or an azaspiroalkyl group having from 6 to 12 ring members that is unsubstituted or substituted by one or more radicals selected from halogen, oxo, lower alkyl and lower alkoxy,
with the proviso that when R₂ represents a group 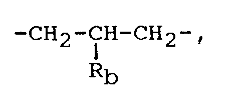 R₃ and R₄ may not form, together with the nitrogen atom carrying them, a piperazine group substituted at the 4-position by a diphenylmethyl radical that is unsubstituted or substituted in the phenyl nuclei by one or more radicals selected from halogen, lower alkyl, lower alkoxy and trifluoromethyl,
wherein:
. the expression "substituted" associated with the term "heterocycle" indicates that the heterocycle is substituted by one or more radicals selected from:
- halogen,
- hydroxy,
- oxo,
- lower alkyl,
- lower alkoxy,
- lower alkoxycarbonyl,
- and the groups -(CH₂)_{n'}-E, 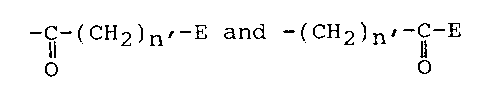 wherein n' represents 0 or an integer from 1 to 4 and E represents a radical selected from aryl, benzhydryl, thienyl, pyrrolyl, pyrrolidinyl, furyl, pyrimidinyl, pyridyl, cycloalkyl and dicycloalkyl-lower alkyl, it being possible for the group E to be unsubstituted or substituted by one or more groups selected from halogen, hydroxy, lower alkyl, lower alkoxy and trifluoromethyl,
. the term "cycloalkyl" represents a cyclic group having from 3 to 8 carbon atoms,
. the term "acyl" represents a lower alkylcarbonyl, arylcarbonyl or aryl-lower alkylcarbonyl group,
. the term "aryl" represents a group selected from phenyl and naphthyl,
. and the terms "lower alkyl" and "lower alkoxy" indicate linear or branched groups containing from 1 to 6 carbon atoms,
their optical isomers, in pure form or in the form of a mixture, and their addition salts with a pharmaceutically acceptable acid or base.

2. Compounds according to claim 1 in which R₃ and R₄, together with the nitrogen atom carrying them, form a piperazine group that is unsubstituted or substituted at the 4-position by a radical selected from:
- lower alkyl,
- lower alkoxy,
- lower alkoxycarbonyl,
- and the groups -(CH₂)_{n'}-E, 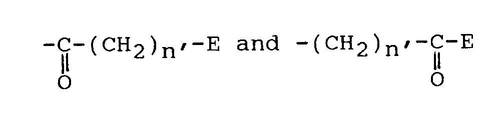 wherein n' represents 0 or an integer from 1 to 4 and E represents a radical selected from aryl, benzhydryl, thienyl, pyrrolyl, pyrrolidinyl, furyl, pyrimidinyl, pyridyl, cycloalkyl and dicycloalkyl-lower alkyl, it being possible for the group E to be unsubstituted or substituted by one or more groups selected from halogen, oxo, hydroxy, lower alkyl, lower alkoxy and trifluoromethyl,
their optical isomers and their addition salts with a pharmaceutically acceptable acid or base.

3. Compounds according to claim 1 in which R₂ represents a group -(CH₂)ₙ- or 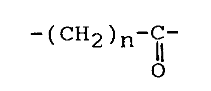 wherein n represents an integer from 1 to 6, it being possible for the groups -(CH₂)ₙ- and 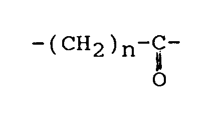 to be substituted in their alkylene moiety by a lower alkyl, aryl or aryl-lower alkyl radical,
their optical isomers and their addition salts with a pharmaceutically acceptable acid or base.

4. Compounds according to claim 1 in which R₂ represents a group 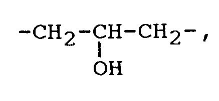 their optical isomers and their addition salts with a pharmaceutically acceptable acid or base.

5. A compound according to claim 1 which is 8-{{3-[4-(2-methoxyphenyl)piperazin-1-yl]-2-hydroxypropyl}oxy}thiochroman, its optical isomers and its addition salts with a pharmaceutically acceptable acid.

6. A compound according to claim 1 which is 8-{{4-[4-(2-methoxyphenyl)piperazin-1-yl]butyl}oxy}thiochroman, and its addition salts with a pharmaceutically acceptable acid.

7. A compound according to claim 1 which is 8-{{4-[4-(3-trifluoromethylphenyl)piperazin-1-yl]butyl}oxy}thiochroman, and its addition salts with a pharmaceutically acceptable acid.

8. A compound according to claim 1 which is 8-{{3-{N,N-di-[4-(7,9-dioxo-8-azaspiro[4.5]decan-8-yl)but-1-yl]amino}-2-hydroxypropyl}oxy}thiochroman, its optical isomers and its addition salts with a pharmaceutically acceptable acid.

9. A compound according to claim 1 which is 8-{[(2-oxo-3-tert.-butyloxazolidin-5-yl)methyl]oxy}thiochroman, its optical isomers and its addition salts with a pharmaceutically acceptable acid.

10. A compound according to claim 1 which is 8-{[4-(7,9-dioxo-8-azaspiro[4.5]decan-8-yl)butyl]oxy}thiochroman, and its addition salts with a pharmaceutically acceptable acid.

11. Process for the preparation of compounds of formula (I) according to claim 1, characterised in that there is used as starting material a thiochromanol of formula (II): 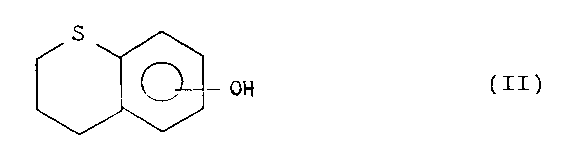 which is reacted:
- either with a dihalogenated compound of formula (IIₐ):
X₁-R'₂-Y'₁ (IIₐ),
in which R'₂ is as defined in claim 1, X₁ represents a halogen atom and Y'₁ represents a halogen atom or, when R'₂ contains a carbonyl function, a (C₁-C₄)-alkoxy radical or a hydroxy radical, to give a compound of formula (II_{b}): 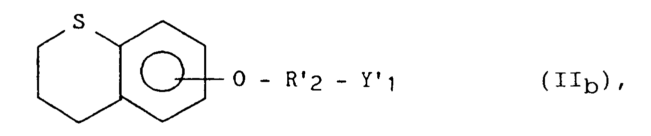 in which R'₂ and Y'₁ are as defined above, which is reacted, after hydrolysis of the ester function when Y₁ represents a (C₁-C₄)-alkoxy radical, with an amine of formula (II_{c}): 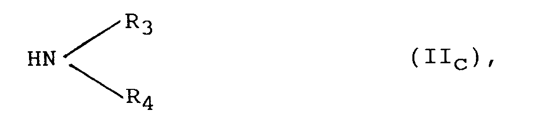 in which R₃ and R₄ are as defined in claim 1, to give a compound of formula (I₁): 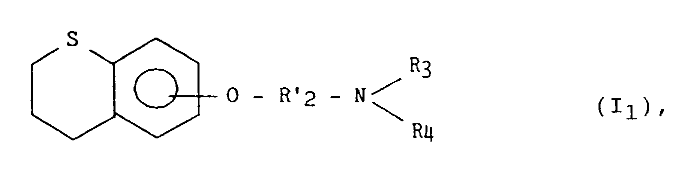 in which R'₂, R₃ and R₄ are as defined above,
which is a particular case of the compounds of formula (I) wherein R₁ represents a group 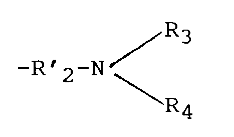 wherein R'₂, R₃ and R₄ are as defined above,
- or with a compound of formula (II_{d}) or (II_{d'}): 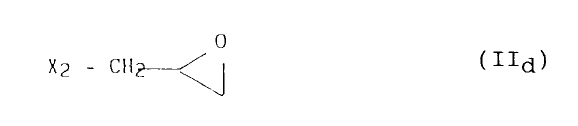
X₂ ―(CH₂)ₚ―CH=CH₂ (II_{d'}),
wherein X₂ represents a halogen atom and p is as defined in formula (I), to give, after treatment with a peracid when a reagent of formula (II_{d'}) has been used, a compound of formula (IIₑ): 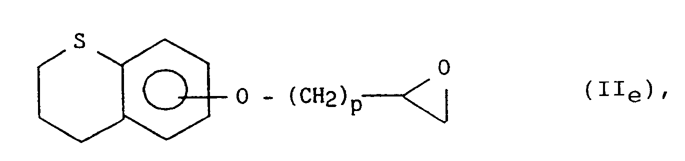 wherein p is as defined above,
which compound of formula (IIₑ) is reacted:
* either with an amine of formula (II_{c}): 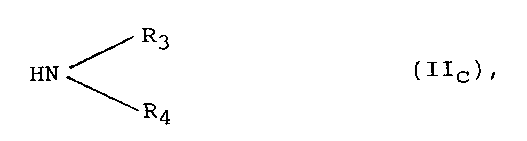 in which R₃ and R₄ are as defined above, to give a compound of formula (I₂): 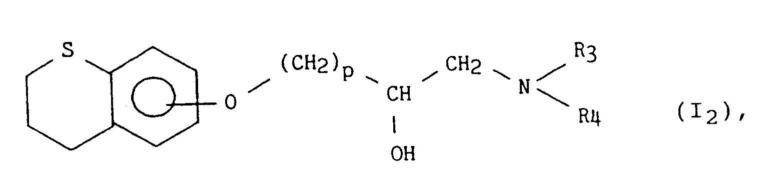 in which p, R₃ and R₄ are as defined above, which is a particular case of the compounds of formula (I) according to claim 1 in which R₁ represents a group: 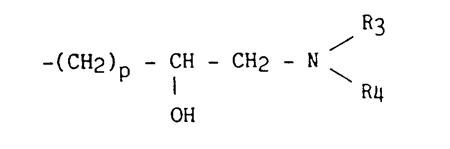 wherein p, R₃ and R₄ are as defined above,
which compounds of formula (I₂) are substituted at the hydroxy group situated in the β-position to the nitrogen atom carrying the radicals R₃ and R₄, by reaction with a compound of formula (II_{f}):
X₃-R_{β} (II_{f}),
in which X₃ represents a halogen atom and R_{β} is a lower alkyl, lower alkylcarbonyl, aryl or aryl-lower alkyl radical, the terms "lower alkyl" and "aryl" being as defined in claim 1, that reaction optionally being accompanied by a step in which the nitrogen carrying R₃ and R₄ is protected, to give a product of formula (I₃): 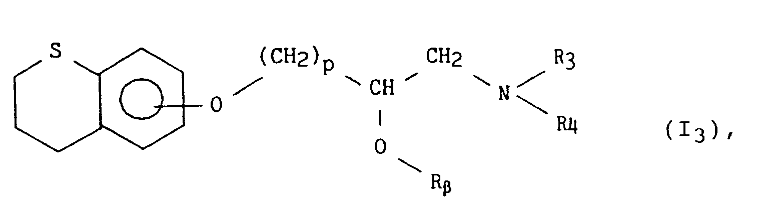 wherein p, R₃, R₄ and R_{β} are as defined above,
which is a particular case of the compounds of the general formula (I) according to claim 1 wherein R₁ represents a group: 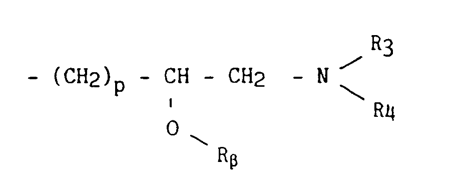 wherein p, R₃, R₄ and R_{β} are as defined above,
* or with sodium azide, to give a compound of formula (II_{g}): 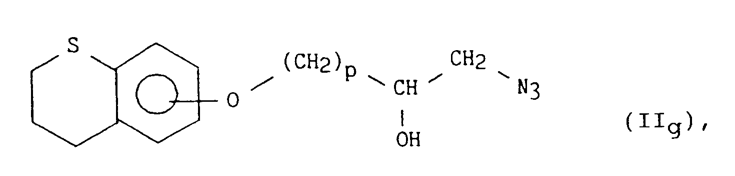 wherein p is as defined above, which is then reacted with ethanol and a Lindlar catalyst in order to obtain a primary amine of formula (I₄): 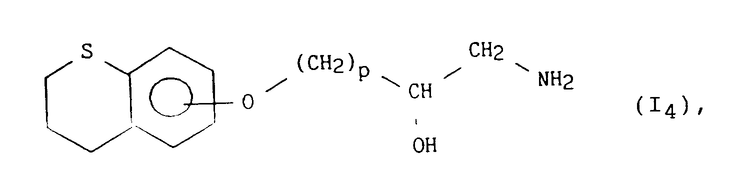 wherein p is as defined above, which is a particular case of the compounds of the general formula (I₂) wherein R₁ represents a group: 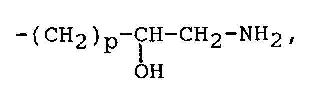 wherein p is as defined above,
which compound of formula (I₄) is reacted with N,N'-carbonyldiimidazole to obtain a compound of formula (I₅): 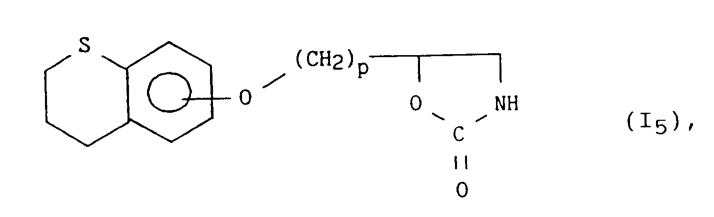 in which p is as defined above, which is a particular case of the compounds of formula (I) according to claim 1 wherein R₁ represents a group of the formula: 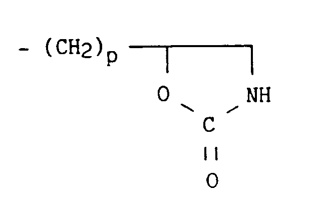 in which p is as defined above,
which compound of formula (I₅) is alkylated, where applicable, by means of a halo-lower alkyl compound of formula (IIₕ):
Rₐ'-X₄ (IIₕ),
wherein Rₐ' is a lower alkyl radical and X₄ is a halogen atom,
to give a compound of formula (I₆): 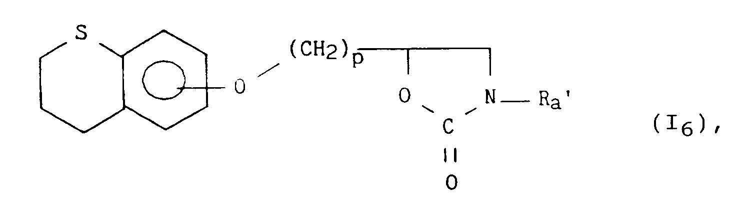 in which p and Rₐ' are as defined above,
which is a particular case of the compounds of formula (I) according to claim 1 wherein R₁ represents a group of the formula: 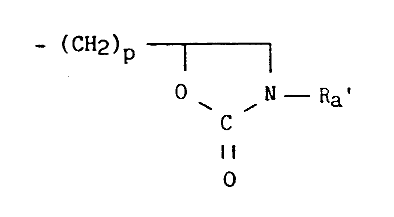 in which p and Rₐ' are as defined above,
the compounds of formula (I₁), (I₂), (I₃), (I₄), (I₅) and (I₆) forming the compounds of formula (I) according to claim 1 in their entirety, which compounds may, if desired, be
- alkylated, if a secondary amine is present, at the nitrogen carrying the substituents R₃ and R₄,
- purified by one or more purification methods selected from crystallisation, chromatography over a silica column, extraction, filtration, and passage over carbon or resin,
- separated, where applicable, in pure form or in the form of a mixture, into their possible optical isomers,
- or converted into a salt with a pharmaceutically acceptable acid or base.

12. Process for the preparation of compounds of formulae (I₇) and (I₇'), which are a particular case of the compounds of formula (I) according to claim 1: 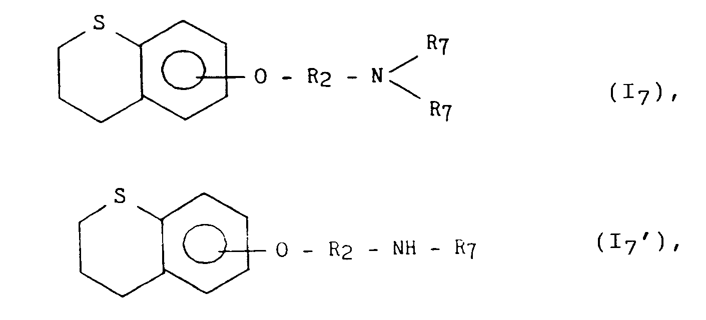 in which R₂ is as defined in claim 1 and R₇ represents:
- a lower alkyl radical that is unsubstituted or substituted by one or more radicals selected from halogen, hydroxy and lower alkoxy,
- a cycloalkyl, cycloalkyl-lower alkyl or di(cycloalkyl)-lower alkyl radical each of which is unsubstituted or substituted in the cycloalkyl moiety/moieties by one or more radicals selected from halogen, oxo, lower alkyl and lower alkoxy,
- an acyl radical that is unsubstituted or substituted by one or more radicals selected from halogen, lower alkyl and lower alkoxy,
- a radical selected from adamantyl, benzopyranyl, benzofuryl and aryl, that radical being unsubstituted or substituted by one or more radicals selected from halogen, lower alkyl, lower alkoxy and trifluoromethyl,
- an aryl-lower alkyl radical that is unsubstituted or substituted in the aryl nucleus by one or more radicals selected from halogen, lower alkyl, lower alkoxy and trifluoromethyl,
- or a radical 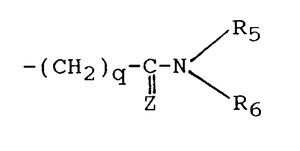 in which q represents 0 or an integer from 1 to 4 and Z represents an oxygen atom or two hydrogen atoms and in which R₅ and R₆ are as defined in claim 1,
the terms "lower alkyl", "lower alkoxy", "cycloalkyl", "acyl" and "aryl" being as defined in claim 1,
characterised in that a compound of formula (I₈): 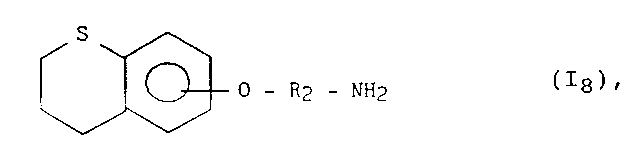 wherein R₂ is as defined above,
is reacted with a halogenated compound of formula (IIᵢ):
X₅-R₇ (IIᵢ),
in which R₇ is as defined above and X₅ represents a halogen atom,
to give, depending on the stoichiometry used, a compound of formula (I₇) or (I₇') as defined above,
which compounds of formulae (I₇) and (I₇') may, if desired, be
- purified by one or more purification methods selected from crystallisation, chromatography over a silica column, extraction, filtration, and passage over carbon or resin,
- separated, where applicable, in pure form or in the form of a mixture, into their possible optical isomers,
- or converted into a salt with a pharmaceutically acceptable acid or base.

13. Process for the preparation of compounds of formula (I₁): 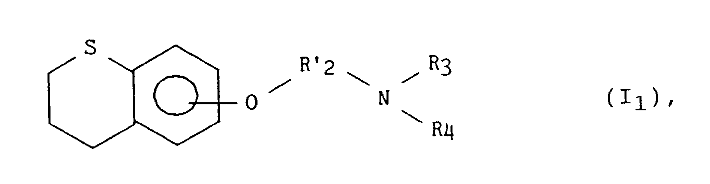 in which R'₂, R₃ and R₄ are as defined in claim 1,
which are a particular case of the compounds of formula (I) according to claim 1 wherein R₁ represents a group of the formula: 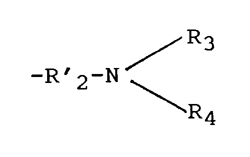 in which R'₂, R₃ and R₄ are as defined above,
characterised in that:
a thiochromanol of formula (II): 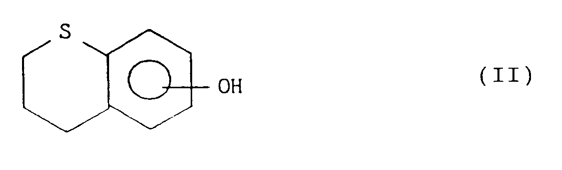 is reacted with a haloalkylamine of formula (IIⱼ): 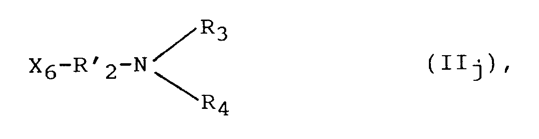 wherein R'₂, R₃ and R₄ are as defined above and X₆ represents a halogen atom, to give a compound of formula (I₁) as defined above,
which compounds of formula (I₁) may, if desired, be
- purified by one or more purification methods selected from crystallisation, chromatography over a silica column, extraction, filtration, and passage over carbon or resin,
- separated, where applicable, in pure form or in the form of a mixture, into their possible optical isomers,
- or converted into a salt with a pharmaceutically acceptable acid or base.

14. Process for the preparation of compounds of formula (I₆), which are a particular case of the compounds of formula (I) according to claim 1: 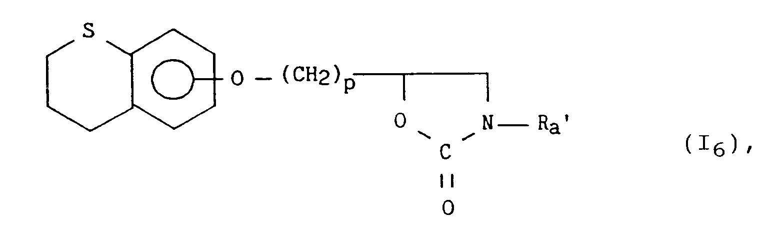 wherein p is as defined in claim 1 and Rₐ' represents a lower alkyl radical,
characterised in that a compound of formula (IIₖ): 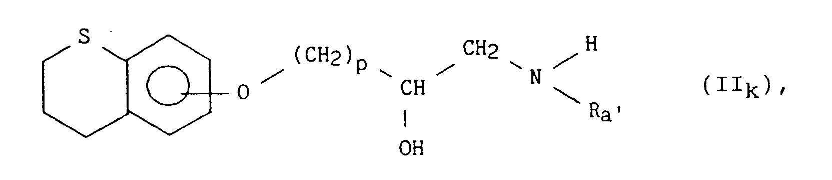 in which p and Rₐ' are as defined above, which is a particular case of the compounds of formula (I) according to claim 1 in which R₁ represents a group 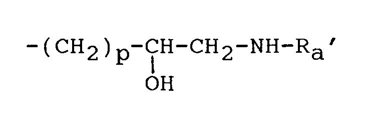 in which Rₐ' and p are as defined above, is reacted with N,N'-carbonyldiimidazole to obtain a compound of formula (I₆) as defined above,
which is a particular case of the compounds of formula (I) according to claim 1 wherein R₁ represents a group of the formula: 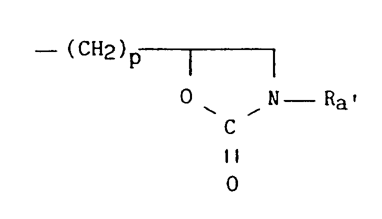 in which Rₐ' and p are as defined above,
which compounds of formula (I₆) may, if desired, be
- purified by one or more purification methods selected from crystallisation, chromatography over a silica column, extraction, filtration, and passage over carbon and/or resin,
- separated, where applicable, in pure form or in the form of a mixture, into their optical isomers.

15. Pharmaceutical composition containing as active ingredient at least one compound according to claim 1 or an addition salt thereof with a pharmaceutically acceptable acid or base, in combination with one or more pharmaceutically acceptable excipients or carriers.

16. Pharmaceutical composition containing as active ingredient at least one compound according to claim 1 or an addition salt thereof with a pharmaceutically acceptable acid or base, in association with at least one known anti-depressant active ingredient, in combination with one or more pharmaceutically acceptable excipients or carriers.

17. Pharmaceutical composition according to either claim 15 or 16 for use in the treatment and prevention of stress, anxiety, depression, psychoses, schizophrenia, pain, and disorders of alimentary and sexual functions.

18. Pharmaceutical composition according to either claim 15 or 16 for use in the treatment of depression.

## Patentansprüche

1. Verbindungen der Formel (I): 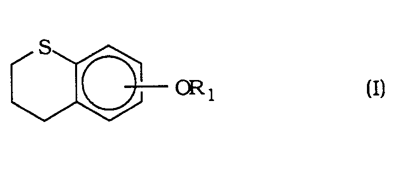 in der:
- R₁:
* eine Gruppe der Formel (A): 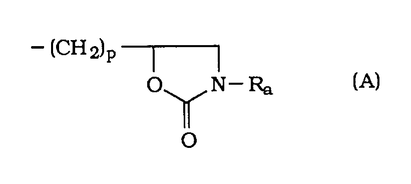 in der Rₐ ein Wasserstoffatom oder eine Niedrigalkylgruppe und p 1, 2 oder 3 bedeuten,
* oder eine Gruppe der Formel (B): 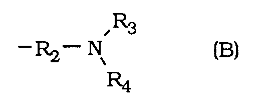 in der
→ R₂ eine Gruppe ausgewählt aus:
- R'₂-, worin R'₂ eine Gruppe ―(CH₂)ₙ― oder 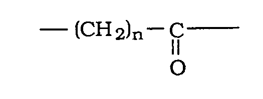 darstellt, worin n eine ganze Zahl mit einem Wert von 1 bis 6 bedeutet, wobei R'₂ nichtsubstituiert oder am Alkylenrest durch eine Niedrigalkylgruppe, Arylgruppe oder Arylniedrigalkylgruppe substituiert ist,
- und 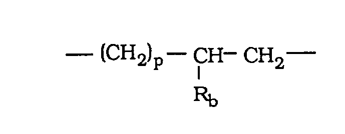 worin p 1, 2 oder 3 und R_{b} eine Hydroxylgruppe, eine Niedrigalkoxygruppe, eine Niedrigalkylcarbonyloxygruppe, eine Aryloxygruppe oder eine (Arylniedrigalkyl)-oxygruppe bedeuten, darstellt, und
→ R₃ und R₄, die gleichartig oder verschieden sein können, jeweils unabhängig voneinander
- ein Wasserstoffatom,
- eine Niedrigalkylgruppe, die nichtsubstituiert oder durch eine oder mehrere Gruppen ausgewählt aus Halogenatomen, Hydroxylgruppen und Niedrigalkoxygruppen substituiert ist,
- eine Cycloalkylgruppe, Cycloalkylniedrigalkylgruppe, Di(cycloalkyl)niedrigalkylgruppe, die nichtsubstituiert oder an den "Cycloalkylresten" durch einen oder mehrere Reste ausgewählt aus Halogenatomen, Oxogruppen, Niedrigalkylgruppen und Niedrigalkoxygruppen substituiert ist,
- eine Acylgruppe, die nichtsubstituiert oder durch eine oder mehrere Gruppen ausgewählt aus Halogenatomen, Niedrigalkylgruppen und Niedrigalkoxygruppen substituiert ist,
- eine aus Adamantyl-, Benzopyranyl-, Benzofuryl- und Arylgruppen ausgewählte Gruppe, wobei diese Gruppe nichtsubstituiert oder durch einen oder mehrere Reste ausgewählt aus Halogenatomen, Hydroxylgruppen, Niedrigalkylgruppen, Niedrigalkoxygruppen und Trifluormethylgruppen substituiert ist,
- eine Arylniedrigalkylgruppe, die nichtsubstituiert oder am Arylrest durch einen oder mehrere Reste ausgewählt aus Halogenatomen, Hydroxylgruppen, Niedrigalkylgruppen, Niedrigalkoxygruppen und Trifluormethylgruppen substituiert ist,
- eine Gruppe 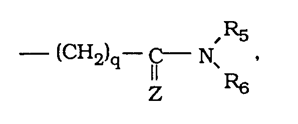 in der q 0 oder eine ganze Zahl mit einem Wert von 1 bis 4 und Z ein Sauerstoffatom oder zwei Wasserstoffatome
und R₅ und R₆ unabhängig voneinander ein Wasserstoffatom, eine Niedrigalkylgruppe, eine Acylgruppe, eine Arylgruppe, die nichtsubstituiert oder durch einen oder mehrere Reste ausgewählt aus Halogenatomen, Hydroxylgruppen, Niedrigalkylgruppen, Niedrigalkoxygruppen und Trifiuormethylgruppen substituiert ist; oder eine Arylniedrigalkylgruppe, die nichtsubstituiert oder am Arylkern durch einen oder mehrere Gruppen ausgewählt aus Halogenatomen, Hydroxylgruppen, Niedrigalkylgruppen, Niedrigalkoxygruppen und Trifluormethylgruppen substituiert ist,
oder R₅ und R₆ gemeinsam mit dem sie tragenden Stickstoffatom entweder einen nichtsubstituierten oder substituierten Heterocyclus ausgewählt aus Pyrrolidin, Piperidin, Piperazin, Morpholin, Thiomorpholin, Homopiperazin, Homopiperidin, Phthalimid und Azabicycloalkyl mit 7 bis 12 Ringgliedern oder (Azaspiroalkyl)-niedrigalkyl, wobei das Azaspiroalkyl-System 6 bis 12 Kohlenstoffatome aufweist und nichtsubstituiert oder durch einen oder mehrere Reste ausgewählt aus Halogenatomen, Oxogruppen und Niedrigalkylgruppen substituiert ist, darstellen,
mit der Maßgabe, daß, wenn R₂ eine Gruppe 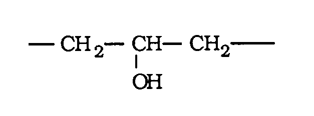 und gleichzeitig R₃ ein Wasserstoffatom darstellen, R₄ nicht ein Wasserstoffatom, eine Niedrigalkylgruppe oder eine Cycloalkylgruppe darstellt,
→ oder R₃ und R₄ mit dem sie tragenden Stickstoffatom:
- entweder einen nichtsubstituierten oder substituierten Heterocyclus ausgewählt aus Pyrrolidin, Piperidin, Piperazin, Morpholin, Thiomorpholin, Homopiperazin, Homopiperidin, Phthalimid und Azabicycloalkyl mit 7 bis 12 Ringgliedern,
mit der Maßgabe, daß, wenn R₂ eine Gruppe -(CH₂)ₙ-, worin n die oben angegebenen Bedeutungen besitzt, darstellt, R₃ und R₄ mit dem sie tragenden Stickstoffatom nicht eine Piperidinogruppe darstellen, die nichtsubstituiert oder durch eine oder mehrere Niedrigalkylgruppen oder Niedrigalkoxygruppen substituiert ist,
- oder eine Azaspiroalkylgruppe mit 6 bis 12 Ringgliedern, die nichtsubstituiert oder durch einen oder mehrere Reste ausgewählt aus Halogenatomen, Oxogruppen, Niedrigalkylgruppen und Niedrigalkoxygruppen substituiert ist,
mit der Maßgabe, daß, wenn R₂ eine Gruppe 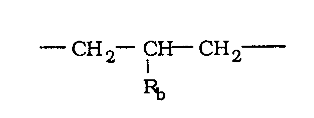 R₃ und R₄ mit dem sie tragenden Stickstoffatom nicht eine Piperazingruppe darstellen, die in der 4-Stellung mit einer Diphenylmethylgruppe substituiert ist, die nichtsubstituiert oder an den Phenylresten durch einen oder mehrere Reste ausgewählt aus Halogenatomen, Niedrigalkylgruppen, Niedrigalkoxygruppen und Trifluormethylgruppen substituiert ist, darstellen,
bedeutet, wobei:
· der mit dem Begriff "Heterocyclus" benutzte Ausdruck "substituiert" bedeutet, daß der Heterocyclus durch einen oder mehrere Reste ausgewählt aus:
- Halogenatomen,
- Hydroxylgruppen,
- Oxogruppen,
- Niedrigalkylgruppen,
- Niedrigalkoxygruppen,
- Niedrigalkoxycarbonylgruppen,
- und Gruppen
―(CH₂)_{n'}―E, 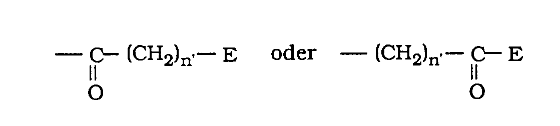 worin n' 0 oder eine ganze Zahl mit einem Wert von 1 bis 4 und E einen Rest ausgewählt aus Aryl-, Benzhydryl-, Thienyl-, Pyrrolyl-, Pyrrolidinyl-, Furyl-, Pyrimidinyl-, Pyridyl-, Cycloalkyl- und Dicycloalkylniedrigalkyl-gruppen darstellen, wobei die Gruppen E nichtsubstituiert oder durch eine oder mehrere Gruppen ausgewählt aus Halogenatomen, Hydroxylgruppen, Niedrigalkylgruppen, Niedrigalkoxygruppen und Trifluormethylgruppen substituiert sein können, substituiert ist,
· der Begriff "Cycloalkyl" eine cyclische Gruppe mit 3 bis 8 Kohlenstoffatomen darstellt,
· der Begriff "Acyl" für eine Niedrigalkylcarbonylgruppe, Arylcarbonylgruppe und Niedrigarylalkylcarbonylgruppe steht,
· der Begriff "Aryl" für eine aus Phenyl- und Naphthylgruppen ausgewählte Gruppe steht, und
· die Begriffe "Niedrigalkylgruppe" und "Niedrigalkoxygruppe" für geradkettige oder verzweigte Gruppen stehen, die 1 bis 6 Kohlenstoffatome aufweisen,
deren optische Isomeren in reiner Form oder in Form einer Mischung, sowie ihre Additionssalze mit einer pharmazeutisch annehmbaren Säure oder Base.

2. Verbindungen nach Anspruch 1, worin R₃ und R₄ mit dem sie tragenden Stickstoffatom einen Piperazinrest bilden, der nichtsubstituiert oder in der 4-Stellung durch einen Rest ausgewählt aus:
- Niedrigalkylgruppen,
- Niedrigalkoxygruppen,
- Niedrigalkoxycarbonylgruppen
- und Gruppen
―(CH₂)_{n'}―E, 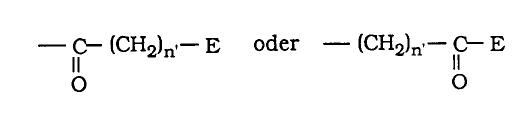 worin n' 0 oder eine ganze Zahl mit einem Wert von 1 bis 4 und E einen Rest ausgewählt aus Aryl-, Benzhydryl-, Thienyl-, Pyrrolyl-, Pyrrolidinyl-, Furyl-, Pyrimidinyl-, Pyridyl-, Cycloalkyl- und Dicycloalkylniedrigalkyl-gruppen darstellen, wobei die Gruppe E nichtsubstituiert oder durch eine oder mehrere Gruppen ausgewählt aus Halogenatomen, Oxogruppen, Hydroxylgruppen, Niedrigalkylgruppen, Niedrigalkoxygruppen und Trifluormethylgruppen substituiert sein kann
deren optische Isomeren und deren Additionssalze mit einer pharmazeutisch annehmbaren Säure oder Base.

3. Verbindungen nach Anspruch 1, worin R₂ eine Gruppe
―(CH₂)ₙ― oder 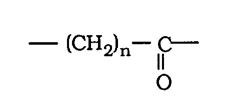 bedeutet, worin n eine ganze Zahl mit einem Wert von 1 bis 6 darstellt und die Gruppen
―(CH₂)ₙ― und 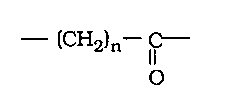 an ihrem Alkylenrest durch eine Niedrigalkylgruppe, eine Arylgruppe oder eine Arylniedrigalkylgruppe substituiert sein können, deren optische Isomeren und deren Additionssalze mit einer pharmazeutisch annehmbaren Säure oder Base.

4. Verbindungen nach Anspruch 1, worin R₂ eine Gruppe 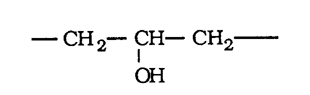 darstellt, deren optische Isomeren sowie deren Additionssalze mit einer pharmazeutisch annehmbaren Säure oder Base.

5. Verbindung nach Anspruch 1, nämlich 8-{{3-[4-(2-Methoxyphenyl)-piperazin-1-yl]-2-hydroxypropyl}-oxy}-thiochroman, dessen optische Isomeren sowie dessen Additionssalze mit einer pharmazeutisch annehmbaren Säure.

6. Verbindung nach Anspruch 1, nämlich 8-{{4-[4-(2-Methoxyphenyl)-piperazin-1-yl]-butyl}-oxy}-thiochroman und dessen Additionssalze mit einer pharmazeutisch annehmbaren Säure.

7. Verbindung nach Anspruch 1, nämlich 8-{{4-[4-(3-Trifluormethylphenyl)-piperazln-1-yl]-butyl}-oxy}-thiochroman sowie dessen Additionssalze mit einer pharmazeutisch annehmbaren Säure.

8. Verbindung nach Anspruch 1, nämlich 8-{{3-{N,N-Di-[4-(7,9-dioxo-8-azaspiro[4,5]decan-8-yl)-but-1-yl]-amino}-2-hydroxypropyl}-oxy}-thiochroman,
dessen optische Isomeren sowie dessen Additionssalze mit einer pharmazeutisch annehmbaren Säure.

9. Verbindung nach Anspruch 1, nämlich 8-{[(2-Oxo-3-tert.-butyl-oxazolidin-5-yl)-methyl]-oxy}-thiochroman sowie dessen optische Isomeren und dessen Additionssalze mit einer pharmazeutisch annehmbaren Säure.

10. Verbindung nachAnspruch 1, nämlich 8-{[4-(7,9-Dioxo-8-azaspiro[4,5]decan-1-yl)-butyl]-oxy}-thiochroman sowie dessen Additionssalze mit einer pharmazeutisch annehmbaren Säure.

11. Verfahren zur Herstellung der Verbindungen der Formel (I) nach Anspruch 1, **dadurch gekennzeichnet**, daß man als Ausgangsmaterial ein Thiochromanol der Formel (II): 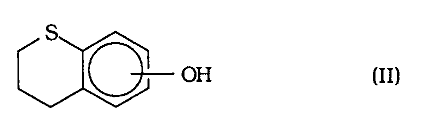 verwendet und dieses:
- entweder mit einer Dihalogenverbindung der Formel (IIₐ):
X₁ - R'₂ - Y'₁ (IIₐ)
in der R'₂ die in Anspruch 1 angegebenen Bedeutungen besitzt, X₁ ein Halogenatom und Y'₁ eine Halogenatom oder eine C₁-C₄-Alkoxygruppe oder eine Hydroxylgruppe, wenn R'₂ eine Carbonylgruppe enthält, bedeuten, umsetzt zur Bildung einer Verbindung der Formel (II_{b}): 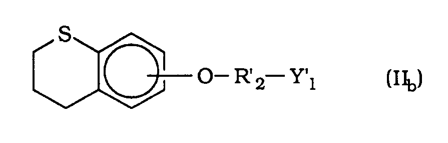 in der R'₂ und Y'₁ die oben angegebenen Bedeutungen besitzen, welche man nach einer Hydrolyse der Esterfunktion, wenn Y₁ eine C₁-C₄-Alkoxygruppe darstellt, mit einem Amin der Formel (II_{c}): 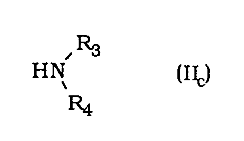 in der R₃ und R₄ die in Anspruch 1 angegebenen Bedeutungen besitzen, umsetzt zur Bildung einer Verbindung der Formel (I₁): 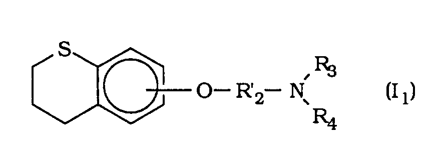 in der R'₂, R₃ und R₄ die oben angegebenen Bedeutungen besitzen, einem Sonderfall der Verbindungen der Formel (I), worin R₁ eine Gruppe 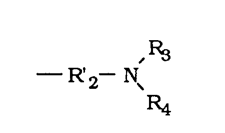 darstellt, worin R'₂, R₃ und R₄ die oben angegebenen Bedeutungen besitzen, - oder mit einer Verbindung der Formel (II_{d}) oder (II_{d'}): 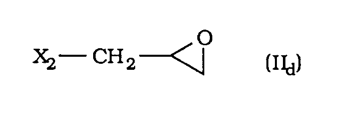
X₂ - (CH₂)ₚ - CH = CH₂ (II_{d'})
worin X₂ ein Halogenatom darstellt und p die bezüglich der Formel (I) angegebenen Bedeutungen besitzt, umsetzt, so daß man nach der Behandlung mit einer Persäure, wenn man ein Reagens der Formel (II_{d'}) verwendet hat, eine Verbindung der Formel (IIₑ): 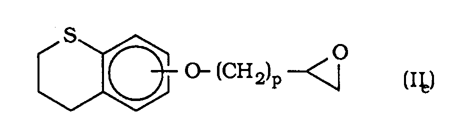 in der p die oben angegebenen Bedeutungen besitzt, erhält,
welche Verbindung der Formel (IIₑ) man:
* entweder mit einem Amin der Formel (II_{c}): 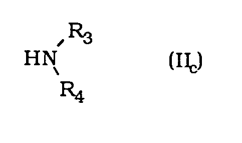 in der R₃ und R₄ die oben angegebenen Bedeutungen besitzen, umsetzt zur Bildung einer Verbindung der Formel (I₂): 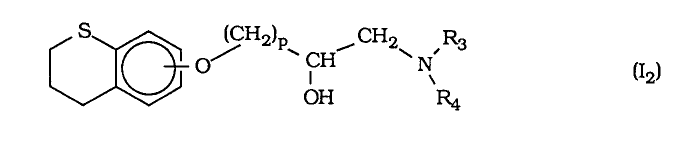 in der p, R₃ und R₄ die oben angegebenen Bedeutungen besitzen, einem Sonderfall der Verbindungen der Formel (I) gemäß Anspruch 1, worin R₁ eine Gruppe: 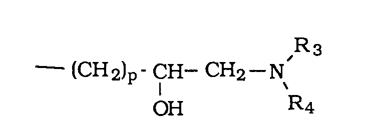 darstellt, worin p, R₃ und R₄ die oben angegebenen Bedeutungen besitzen,
welche Verbindungen der Formel (I₂) man an der Hydroxylgruppe in der β-Stellung zu dem Stickstoffatom, das die Gruppe R₃ und R₄ trägt, durch Reaktion mit einer Verbindung der Formel (II_{f}):
X₃ - R_{β} (II_{f})
worin X₃ ein Halogenatom und R_{β} eine Niedrigalkylgruppe, Niedrigalkylcarbonylgruppe, Arylgruppe oder Arylniedrigalkylgruppe bedeuten, wobei die Begriffe "Niedrigalkylgruppe" und "Arylgruppe" die in Anspruch 1 angegebenen Bedeutungen besitzen, welche Reaktion gegebenenfalls von einer Stufe zum Schützen des Stickstoffatoms, das die Gruppen R₃ und R₄ trägt, substituiert, so daß man ein Produkt der Formel (I₃) erhält: 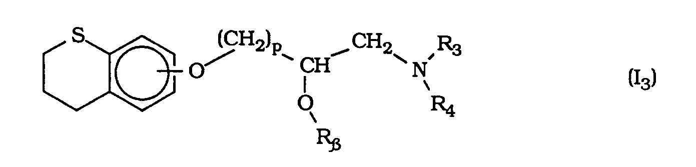 worin p, R₃ R₄ und R_{β} die oben angegebenen Bedeutungen besitzen, einem Sonderfall der Verbindungen der allgemeinen Formel (I) gemäß Anspruch 1, worin R₁ eine Gruppe: 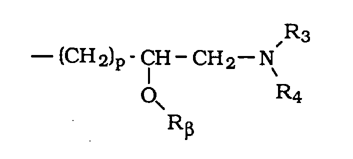 darstellt, in der p, R₃, R₄ und R_{β} die oben angegebenen Bedeutungen besitzen,
* oder mit Natriumazid umsetzt zur Bildung einer Verbindung der Formel (II_{g}): 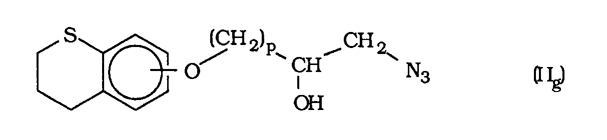 in der p die oben angegebenen Bedeutungen besitzt, welche man anschließend mit Ethanol und einem Lindlar-Katalysator umsetzt zur Bildung eines primären Amins der Formel (I₄): 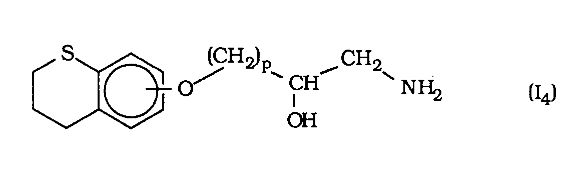 in der p die oben angegebenen Bedeutungen besitzt, einem Sonderfall der Verbindungen der allgemeinen Formel (I₂), worin R₁ eine Gruppe: 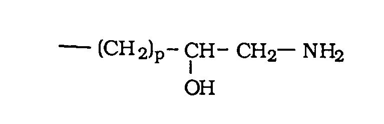 darstellt, worin p die oben angegebenen Bedeutungen besitzt, welche Verbindung der Formel (I₄) man mit N,N'-Carbonyldiimidazol umsetzt zur Bildung einer Verbindung der Formel (I₅): 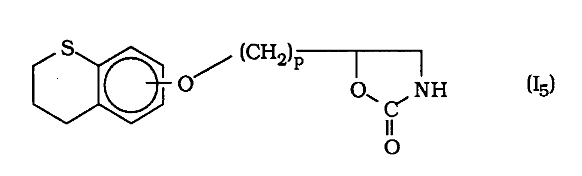 in der p die oben angegebenen Bedeutungen besitzt, einem Sonderfall der Verbindungen der Formel (I) gemäß Anspruch 1, worin R₁ eine Gruppe der Formel: 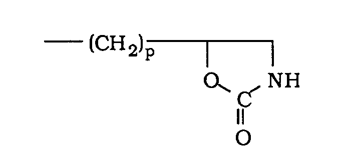 darstellt, in der p die oben angegebenen Bedeutungen besitzt, welche man gegebenenfalls mit Hilfe eines Niedrigalkylhalogenids der Formel (IIₕ):
Rₐ' - X₄ (IIₕ)
in der Rₐ' eine Niedrlgalkylgruppe und X₄ ein Halogenatom darstellen, alkyliert
zur Bildung einer Verbindung der Formel (I₆): 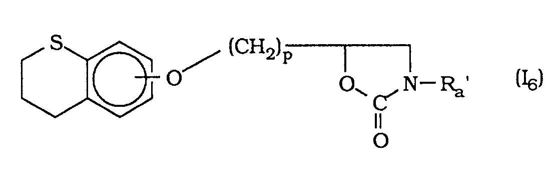 in der p und Rₐ' die oben angegebenen Bedeutungen besitzen, einem Sonderfall der Verbindungen der Formel (I) nach Anspruch 1, worin R₁ eine Gruppe der Formel: 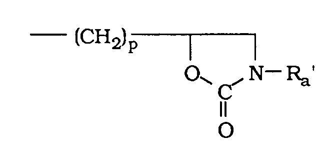 darstellt, worin p und Rₐ' die oben angegebenen Bedeutungen besitzen,
welche Verbindungen der Formeln (I₁), (I₂), (I₃), (I₄), (I₅) und (I₆) gemeinsam die Verbindungen der Formel (I) nach Anspruch 1 bilden, welche gewünschtenfalls,
- wenn ein sekundäres Amin vorliegt, am die Substituenten R₃ und R₄ tragenden Stickstoffatom alkyliert werden können,
- mit Hilfe einer oder mehrerer Reinigungsmethoden, ausgewählt aus Kristallisation, Säulenchromatographie über Siliciumdioxid, Extraktion, Filtration und Überführung über Aktivkohle oder ein Harz, gereinigt werden können,
- gegebenenfalls in ihre eventuellen optischen Isomeren in reiner Form oder in Form einer Mischung aufgetrennt werden können,
- oder mit einer pharmazeutisch annehmbaren Säure oder Base in die Salze überführt werden können.

12. Verfahren zur Herstellung der Verbindungen der Formeln (I₇) und (I_{7'}), einem Sonderfall der Verbindungen der Formel (I) nach Anspruch 1: 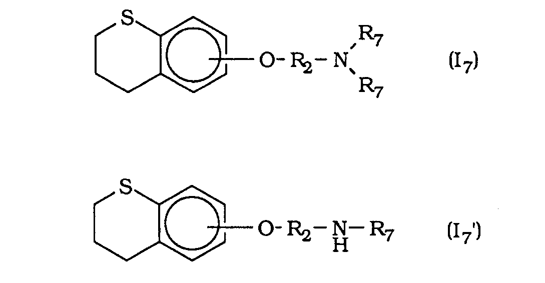 in denen R₂ die in Anspruch 1 angegebenen Bedeutungen besitzt und R₇:
- eine Niedrigalkylgruppe, die nichtsubstituiert oder durch einen oder mehrere Reste, ausgewählt aus Halogenatomen, Hydroxylgruppen und Nledrigalkoxygruppen, substituiert ist,
- eine Cycloalkylgruppe, Cycloalkylniedrigalkylgruppe, Di(cycloalkyl)niedrlgalkylgruppe, die nichtsubstituiert oder an den "Cycloalkylresten" durch einen oder mehrere Reste, ausgewählt aus Halogenatomen, Oxogruppen, Niedrigalkylgruppen und Niedrigalkoxygruppen, substituiert ist,
- eine Acylgruppe, die nichtsubstituiert oder durch einen oder mehrere Reste, ausgewählt aus Halogenatomen, Niedrigalkylgruppen und Niedrigalkoxygruppen, substituiert ist,
- eine aus Adamantyl-, Benzopyranyl-, Benzofuryl- und Arylgruppen ausgewählte Gruppe, die nichtsubstituiert oder durch einen oder mehrere Reste, ausgewählt aus Halogenatomen, Niedrigalkylgruppen, Niedrigalkoxygruppen und Trifluormethylgruppen, substituiert ist,
- eine Arylniedrigalkylgruppe, die nichtsubstituiert oder am Arylkern durch einen oder mehrere Reste, ausgewählt aus Halogenatomen, Niedrigalkylgruppen, Niedrigalkoxygruppen und Trifluormethylgruppen, substituiert ist,
- oder eine Gruppe 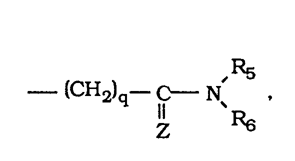 in der q 0 oder eine ganze Zahl mit einem Wert von 1 bis 4 und Z ein Sauerstoffatom oder 2 Wasserstoffatome bedeuten
und R₅ und R₆ die In Anspruch 1 angegebenen Bedeutungen besitzen, bedeuten,
wobei die Begriffe "Niedrigalkylgruppe", "Niedrigalkoxygruppe", "Cycloalkylgruppe", "Acylgruppe" und "Arylgruppe" die in Anspruch 1 angegebenen Bedeutungen besitzen,
**dadurch gekennzeichnet**, daß man eine Verbindung der Formel (I₈): 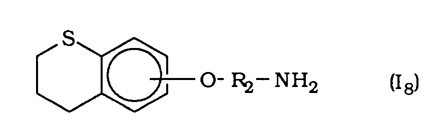 in der R₂ die oben angegebenen Bedeutungen besitzt,
mit einer Halogenverbindung der Formel (IIᵢ):
X₅ - R₇ (IIᵢ)
in der R₇ die oben angegebenen Bedeutungen besitzt und X₅ ein Halogenatom darstellt, umsetzt,
so daß man in Abhängigkeit von der angewandten Stöchiometrie eine Verbindung der Formel (I₇) oder der Formel (I₇'), wie sie oben definiert worden sind, erhält, welche Verbindungen der Formeln (I₇) und (I₇') gewünschtenfalls
- mit Hilfe einer oder mehrerer Reinigungsmethoden, ausgewählt aus Kristallisation, Säulenchromatographie über Siliciumdioxid, Extraktion, Filtration und Überführung über Aktivkohle oder ein Harz, gereinigt werden können,
- gegebenenfalls in ihre eventuellen optischen Isomeren in reiner Form oder in Form einer Mischung aufgetrennt werden können,
- oder mit einer pharmazeutisch annehmbaren Säure oder Base in die Salze überführt werden können.

13. Verfahren zur Herstellung der Verbindungen der Formel (I₁): 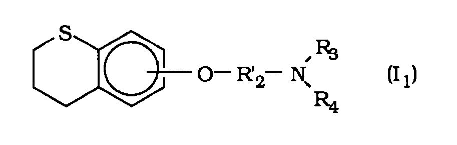 in der R'₂, R₃ und R₄ die in Anspruch 1 angegebenen Bedeutungen besitzen, einem Sonderfall der Verbindungen der Formel (I) nach Anspruch 1, worin R₁ eine Gruppe der Formel darstellt: 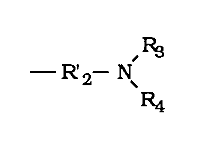 in der R'₂, R₃ und R₄ die oben angegebenen Bedeutungen besitzen,
**dadurch gekennzeichnet**, daß man:
ein Thiochromanol der Formel (II): 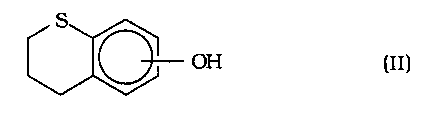 mit einem Halogenalkylamin der Formel (IIⱼ): 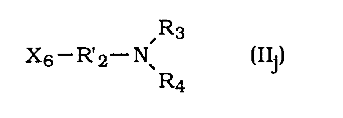 in der R'₂, R₃ und R₄ die oben angegebenen Bedeutungen besitzen und X₆ ein Halogenatom darstellt, umsetzt zur Bildung einer Verbindung der Formel (I₁), wie sie oben definiert worden ist,
welche Verbindungen der Formel (I₁) gegebenenfalls
- mit Hilfe einer oder mehrerer Reinigungsmethoden, ausgewählt aus Kristallisation, Säulenchromatographie über Siliciumdioxid, Extraktion, Filtration und Überführung über Aktivkohle oder ein Harz, gereinigt werden können,
- gegebenenfalls in ihre eventuellen optischen Isomeren in reiner Form oder in Form einer Mischung aufgetrennt werden können,
- oder mit einer pharmazeutisch annehmbaren Säure oder Base in ihre Salze überführt werden können.

14. Verfahren zur Herstellung der Verbindungen der Formel (I₆), einem Sonderfall der Verbindungen der Formel (I) nach Anspruch 1: 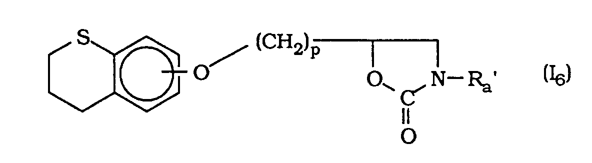 in der p die in Anspruch 1 angegebenen Bedeutungen besitzt und Rₐ' eine Niedrigalkylgruppe bedeutet,
**dadurch gekennzeichnet**, daß man eine Verbindung der Formel (IIₖ): 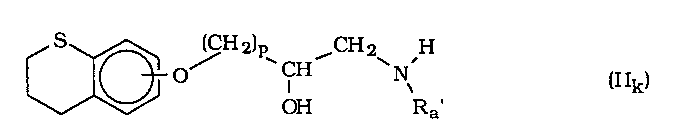 in der p und Rₐ' die oben angegebenen Bedeutungen besitzen, einem Sonderfall der Verbindungen der Formel (I) nach Anspruch 1, worin R₁ eine Gruppe 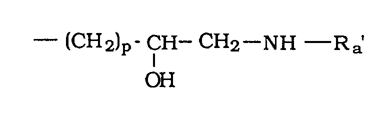 darstellt, worin Rₐ' und p die oben angegebenen Bedeutungen besitzen, mit N,N'-Carbonyldiimidazol umsetzt zur Bildung einer Verbindung der Formel (I₆), wie sie oben definiert worden ist, einem Sonderfall der Verbindungen der Formel (I) nach Anspruch 1, worin R₁ eine Gruppe der Formel darstellt: 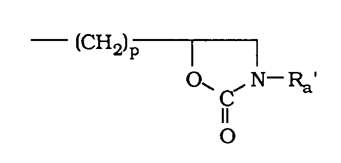 in der Rₐ' und p die oben angegebenen Bedeutungen besitzen,
welche Verbindungen der Formel (I₆) gewünschtenfalls
- mit einer oder mehreren Reinigungsmethoden, ausgewählt aus Kristallisation, Säulenchromatographie über Siliciumdioxid, Extraktion, Filtration und Überführung über Aktivkohle und/oder ein Harz, gereinigt werden können,
- oder gegebenenfalls in ihre optischen Isomeren in reiner Form oder in Form einer Mischung aufgetrennt werden können.

15. Pharmazeutische Zubereitung enthaltend als Wirkstoff mindestens eine Verbindung nach Anspruch 1 oder eines ihrer Additionssalze mit einer pharmazeutisch annehmbaren Säure oder Base in Kombination mit einem oder mehreren pharmazeutisch annehmbaren Trägermaterialien oder Bindemitteln.

16. Pharmazeutische Zubereitung enthaltend als Wirkstoff mindestens eine Verbindung nach Anspruch 1 oder eines ihrer Additionssalze mit einer pharmazeutisch annehmbaren Säure oder Base in Kombination mit mindestens einem bekannten Antidepressivum in Kombination mit einem oder mehreren pharmazeutisch annehmbaren Trägermaterialien oder Bindemitteln.

17. Pharmazeutische Zubereitung nach den Ansprüchen 15 oder 16 geeignet für die Behandlung und die Vorbeugung von Streß, der Angst, der Depression, von Psychosen, der Schizophrenie, von Schmerzen und von Störungen des Ernäherungs-und Sexualverhaltens.

18. Pharmazeutische Zubereitung nach Ansprüchen 15 oder 16 geeignet für die Behandlung der Depression.
